# EUROPEAN PATENT SPECIFICATION

(11) **EP 0 578 775 B1**
(45) Date of publication and mention of the grant of the patent: **27.09.1995**
(21) Application number: 92910842.1
(22) Date of filing: 31.03.1992
(51) Int. Cl.: A61M 5/142, A61M 5/14, H01R 13/629

(54) **AMBULATORY FLUID DELIVERY SYSTEM**
BEWEGLICHES FLÜSSIGKEITSABGABESYSTEM
SYSTEME DE PERFUSION AMBULATOIRE

(30) Priority: 03.04.1991 US 679886; 08.10.1991 US 774014; 08.01.1992 US 818194; 08.01.1992 US 818009; 09.01.1992 US 819300
(43) Date of publication of application: 19.01.1994
(73) Proprietor: SHERWOOD MEDICAL COMPANY, St. Louis, MO 63103-1642 (US)
(72) Inventor: SUNDERLAND, Richard, Arthur, St. Charles, MO 63303 (US); DENO, Frederick, Brownville, NY 13515 (US); GAHN, Gerald, Steven, St. Louis, MO 63021 (US); LANE, John, Andrew, Watertown, NY 13601 (US); LEWIS, Thomas, Gene, Fairview Heights, IL 62208 (US); SCHRADER, Eugene, Francis, St. Louis, MO 63110 (US); YERLIKAYA, Denis, Y., Des Peres, MO 63131 (US)
(74) Representative: Chettle, Adrian John
(86) International application number: US9202619
(87) International publication number: WO9217226

(56) References cited:
- EP-A- 0 039 044
- WO-A-82/03254
- WO-A-90/07947
- DE-A- 3 606 930
- FR-A- 2 162 241
- US-A- 4 688 595

## Description

### TECHNICAL FIELD

### 1. Field of the Invention

This invention relates generally to a fluid delivery system. More specifically, the present invention relates to a support device used as a part of an ambulatory fluid delivery system for supporting and protecting the pump and a fluid delivery set, a connector cable for recharging the pump when mounted to the support device, and a carrying case for ambulatory use of the system.

### 2. Description of the Prior Art

It is common for patient's having certain medical problems to require periodic premeasured infusions of fluid, such as medicaments or nutrients, into their bodies. Examples of such patients are those who may require nutrients to be delivered directly into their digestive tract periodically over long periods of time, or cancer patients who require exacting amounts of medication to be delivered intravenously at precise intervals.

In the past, such patients required hospitalization for the time necessary to infuse the nutrients or medicaments, in order to allow medical personnel to perform the infusions at the proper time and in the proper amounts. Such a procedure was extremely time consuming to the patient and also the hospital personnel, and included the potential of human error in calculation of infusion dosages and injection time intervals.

An improvement on the above procedure has been to employ a programmable pump to insure that the patient receives the proper infusion dosage at the proper time period, thus relieving medical personnel from constant monitoring of the patient, and from worrying about infusion amounts and time tables. Although the programmable pump greatly relieves medical personnel of time consuming care to the patient, the patient nevertheless remains bound to the hospital bed during the prolonged infusion periods.

A further improvement has been to develop an infusion system which can not only automatically infuse preset volumes of fluid into the patient on a predetermined time table, but also allow the patient to be ambulatory. U.S. Patent No. 4,657,486 to Stemple et al., U.S. Patent No. 4,397,639 to Eschweiler et al., and U.S. Patent No. 4,416,595 to Cromie, are exemplary of portable infusion systems of this type. Each discloses a portable infusion device which is automatically operable at selected time intervals to inject accurate amounts of fluid medication into a patient's body, and is also sufficiently compact and portable to allow the patient to be ambulatory during the infusion procedure.

U.S. Patent No. 4,688,595 to Srebnik et al. is also exemplary of fluid delivery systems of this type, and is considered to represent the closest prior art with regard to the present invention. Srebnik discloses a delivery system which includes an integrally molded platform to which elements of the delivery system, i.e., the pump, the fluid container, etc. can be connected. The platform allows the entire fluid delivery system to be transportable as a unit and makes it possible for the patient to move about without the inconvenience of transporting a more cumbersome apparatus such as a prior art type infusion system which was commonly affixed to a pole mounted on wheels.

Although there have been improvements in portable fluid delivery systems in the past, there nevertheless remain several inadequacies. First, the prior art fluid infusion systems generally include a programmable pump, and a fluid delivery set comprising a fluid container, tubing, pinch clamp, drip chamber, etc., all connected as an integral unit. The container of the fluid delivery sets may be a flexible bag, a rigid glass or plastic bottle or a burette. Sometimes these standard fluid delivery sets (intended for non-ambulatory use) include rather long tubular extensions to allow the fluid container to be placed on an infusion pole while the distal end of the tube can be attached to a bed ridden or non-ambulatory patient. These sets are generally ill suited for placement in a portable device such as that described by Srebnik et al., because the portable system requires significantly shorter tubing extension to properly operate. The excess tubing becomes cumbersome and inhibitive of proper operation of the system and often becomes occluded or pinched off during ambulatory use. Often, such prior art ambulatory systems have required a unique "non-standard" tubing design in order to allow the fluid delivery set to be properly attached to the pump. Since the "non-standard" ambulatory sets (such as shown by Stemple et al.) are generally unsuitable for use on standard non-ambulatory systems, it has been necessary for hospitals and other medical facilities to stock "non-standard" fluid delivery sets for use in ambulatory-type systems, and standard sets for all other uses.

### DISCLOSURE OF INVENTION

Briefly, and in general terms, the present invention provides for ambulatory use of a "standard" fluid delivery set, of a fluid delivery system, while at the same time provides for reliable prevention of kinking or occlusion of excess tubing and other inadvertent damage to the system.

It is an object of the present invention to provide a portable fluid delivery system which is designed to accommodate standard fluid delivery sets commonly intended for non-ambulatory use.

It is another object of the present invention to provide a fluid delivery system which is designed to avoid occlusions or damage to the tubing of the fluid delivery sets.

It is another object of the present invention to provide a support device for a fluid delivery system which will allow use of standard fluid delivery sets (designed for non-ambulatory use) thereon and which will protect and avoid occlusion of any excess tubing therein.

It is another object of the present invention to provide a fluid delivery system which includes a support device which is readily adaptable for use with soft bag, blow molded bottle, glass bottle, or burette-type fluid containers of fluid delivery sets.

It is a further object of the present invention to provide a fluid delivery system including a support device which allows for the pump of the fluid delivery system to be readily attached or detached therefrom.

It is another object of the present invention to provide a support device which can be used either free-standing, attached to an infusion pole, or enclosed in a carrying case as part of the fluid delivery system.

It is another object of the present invention to provide a support device which can signal the pump of a fluid delivery system to allow the pump to modify its operation depending on whether or not it is intended to be used in an ambulatory or non-ambulatory manner.

It is further an object of the present invention to provide a support device which will signal the pump mounted therein to compensate for the changes in fluid pressure within the fluid delivery set due to the relative position of the fluid reservoir with respect to the infusion control member on the pump.

It is another object of the present invention to provide a support device and carrying case for a fluid delivery system which will allow use of standard fluid delivery sets (designed for non-ambulatory use) therein and which will protect and avoid occlusion of any excess tubing.

It is another object of the present invention to provide a connector cable which can attach directly to a support device of a fluid delivery system such that subsequent mounting of an infusion pump to the support device will automatically cause connection of the pump to the connector cable.

It is a further object of the present invention to provide a connector cable having a unique connector end characterized by its ability to simultaneously be affixed to a support device in proper position for subsequent electrical connection with the pump as it is mounted to the support device for use.

These and other objects and advantages of the present invention are realized in a specific preferred embodiment thereof, disclosed herein for purposes of example and not by way of limitation, which comprises a support device formed of a rigid body having a first compartment for receiving and locking a standard infusion pump in place therein, and a second, a generically-shaped compartment for receiving and retaining a container of a fluid delivery set in a fixed position relative to the pump. The support device also includes a third compartment formed as an elongated channel extending around a substantial portion of the perimeter of the rigid body into which the tubing of the fluid delivery set can be inserted. The elongate channel is designed to approximately match the length of the tubing included on a "standard" fluid delivery set between the container and the pump to protect the tubing against kinking or occlusion along its entire length. The rigid body also includes straps, brackets, and clamps which are strategically positioned to provide maximum support for any one of several types of containers, such as soft bags, glass bottles, blow molded plastic bottles, burettes, etc.

The support device also includes a sensed means such as a magnet embedded in the pump compartment which can signal a sensor, such as a magnetic field sensor located in the pump, which can detect the sensed means when the pump is properly mounted within the pump compartment of the device. When the sensor detects the magnet, the input from the sensor to the pump causes the pump to select a modified control program which adjusts the operation of the motor within the pump to compensate for variations in fluid pressure within the fluid delivery set caused by ambulatory use of the device.

The support device is preferably designed to be insertable into a carrying case for ambulatory use. However, the rigid body also includes a base which can be used to support the entire fluid delivery system in a free standing manner on a horizontal surface, and which may include an extendable leg to increase the stability thereof during such use. Alternatively, the rigid body may include a strap which allows it to be suspended from a standard infusion pole if desired.

The carrying case is preferably designed to include an extendible "chimney" which allows the carrying case to be adapted for use with fluid sets having large fluid containers which require the clamp of the support device to be moved to an extended position. The extensible chimney is formed of a plurality of flaps which can be folded into a closed, non-extended, position when not in use, and can be unfolded into an extended position when needed to wrap around and encase an extension of the support device when holding a large fluid container.

The fluid delivery system may also be used in conjunction with an elongated electrical connector cable having a first connector on one end thereof adapted for attachment to the ambulatory support device in proper placement for simultaneous attachment to the rechargeable pump, and a second connector on the opposite end thereof adapted for attachment to a charger unit. The first connector includes a primary locking member having a pair of resilient locking fingers which are insertable into a connector port in the support device. During insertion, the fingers resiliently deflect about a pair of locking tabs protruding into the connector port which lock the first connector in place in the support device. An electrical connection member also formed as part of the first connector is positioned relative to the primary locking member such that it is positioned within the pump compartment of the support device at a location predetermined to cause matching of the electrical connector to the recharge receptacle of the pump whenever the pump is properly mounted in the support device. The second connector of the connector cable is attachable to a charger unit, thus allowing recharging of the pump while mounted within the support device.

These and other objects and advantages of the present invention will become apparent from the following more detailed description, when taken in conjunction with the accompanying drawings in which like elements are identified with like numerals throughout.

### BRIEF DESCRIPTION OF DRAWINGS

Figure 1 is a front view of a support device of a fluid delivery system made in accordance with the principals of the present invention;
Figure 2 is a right side view of the support device of Figure 1;
Figure 3 is a left side view of the support device of Figure 1;
Figure 4 is a rear view of the support device shown in Figure 1;
Figure 5 is a front view of the support device as shown in Figure 1, including a pump and a fluid infusion set with a rigid plastic bottle attached thereto for operation;
Figure 6 is a right side view of the support device and attached fluid delivery set as shown in Figure 5;
Figure 7 is a left side view of the support device and attached pump and fluid delivery set as shown in Figure 5;
Figure 8 is a cross-sectional view taken along line VIII-VIII of Figure 2 showing a preferred embodiment of the pump locking mechanism of the present invention;
Figures 9-12 show a preferred embodiment of the saddle bracket of the present invention;
Figure 13 is a cross-sectional view taken along line XIII-XIII of Figure 1 showing the attachment of the saddle bracket to the support device of the present invention;
Figures 14-16 show the support device of the present invention as shown in Figures 1-3 respectively, with a fluid delivery set having a flexible bag-type fluid container attached thereto for operation;
Figures 17-18 show a preferred embodiment of the inner clamp jaw of the lid clamp of the present invention;
Figures 19-20 show a preferred embodiment of the outer clamp jaw of the lid clamp of the present invention;
Figure 21 is a top view of the support device of the present invention as shown in Figure 1;
Figures 22-24 show a front view, left side view, and rear view respectively, of the support device of the present invention with the fluid container lid clamp thereof extended for use of the device with fluid delivery sets which include large fluid containers;
Figures 25-27 show a preferred embodiment of a locking mechanism for the extension clamp of the support device;
Figure 28 is a cross-sectional view taken along line XXVIII-XXVIII of Figure 4 showing the operation of the lid clamp locking mechanism of the support device;
Figure 29 is a cross-sectional view taken along line XXIX-XXIX of Figure 5 showing the tubing of a fluid delivery set located in a preferred embodiment of the tubing compartment of the support device of the present invention;
Figure 30 is a bottom view of the support device of the present invention as shown in Figure 1 with the rotatable support leg thereof as rotated to its extended position being shown in dashed lines;
Figures 31-32 show a preferred embodiment of the rotatable support leg of the support device;
Figure 33 is a cross-sectional view taken along lines XXXIII-XXXIII of Figure 30 showing the rotatable support leg of the support device of the present invention;
Figure 34 is a front view of a carrying case of the fluid delivery system made in accordance with the principles of the present invention;
Figure 35 is a right side view of the carrying case of Figure 34;
Figure 36 is a top view of the carrying case of Figure 34;
Figure 37 is a front view of the preferred embodiment of the carrying case of the fluid delivery system of the present invention showing the extensible chimney thereof in its retracted position;
Figure 38 is a front view of the carrying case of the present invention showing the chimney thereof in its extended position;
Figure 39 is a front view of a preferred embodiment of a fluid infusion pump useable with the fluid delivery system of the present invention;
Figure 40 is a right side view of the pump of Figure 39;
Figure 41 is a top view of the pump of Figure 39;
Figure 42 is a timing diagram illustrating various signals used in conjunction with the fluid delivery pump of the present invention;
Figure 43 is a perspective view of a connector cable made in accordance with the principles of the present invention;
Figure 44 is a top view of a first end of the connector cable showing the first connector and the primary locking member thereof;
Figure 45 is a bottom view of a first connector as shown in Figure 46 showing the location of the electrical connection member thereof relative to the primary locking member;
Figure 46 is a side view of the first connector as shown in Figure 44;
Figure 47 is a front view of the first connector as shown in Figure 44;
Figure 48 is a top view of a second connector of the connector cable of the present invention, with the electrical pins thereof being shown in dashed lines;
Figure 49 is a bottom view of the second connector of Figure 48;
Figure 50 is a side view of the second connector of Figure 48;
Figure 51 is a rear view of a support device to which the first connector of the connector has been attached;
Figure 52 is a front view of the support device with a partial cutaway view of the pump compartment thereof showing the connection of the connector cable thereto; and
Figure 53 is a side view of the support device of Figure 51 showing the connector positioned in the connector port within the pump compartment thereof.

### MODES FOR CARRYING OUT THE INVENTION

As shown in the exemplary drawings for the purposes of illustration, an embodiment of a support device made in accordance with the principles of the present invention, referred to generally by the reference numeral 10, is provided for convenient ambulatory support of a standard (non-ambulatory type) fluid set and infusion pump of a fluid delivery system.

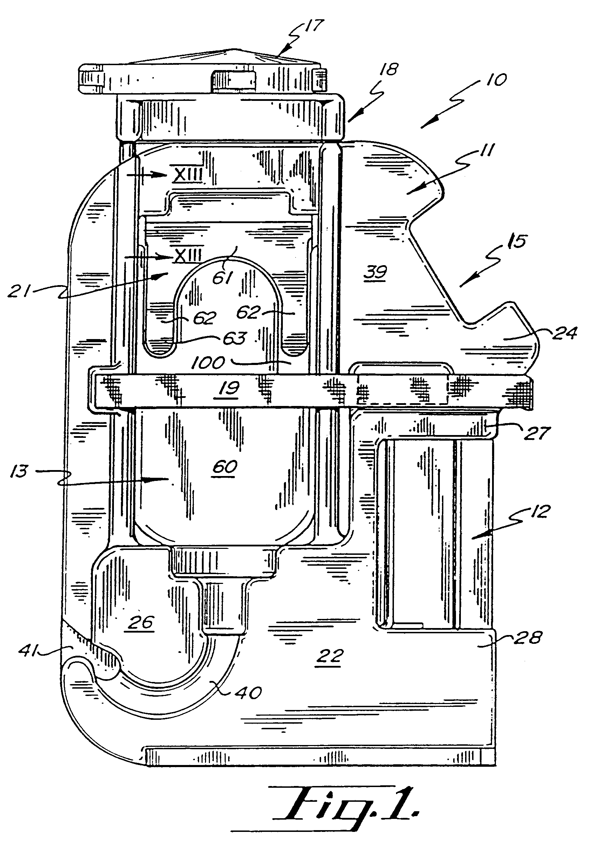

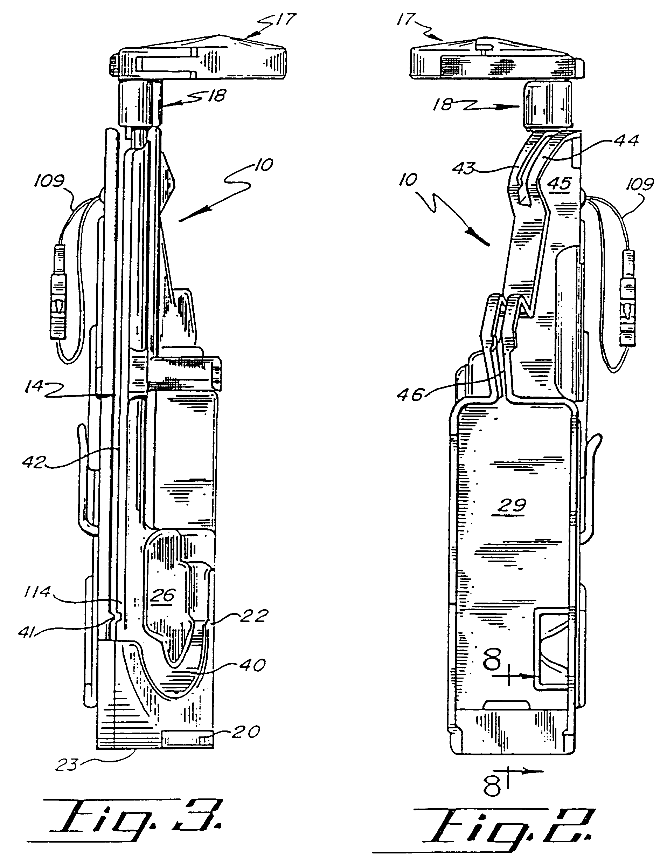

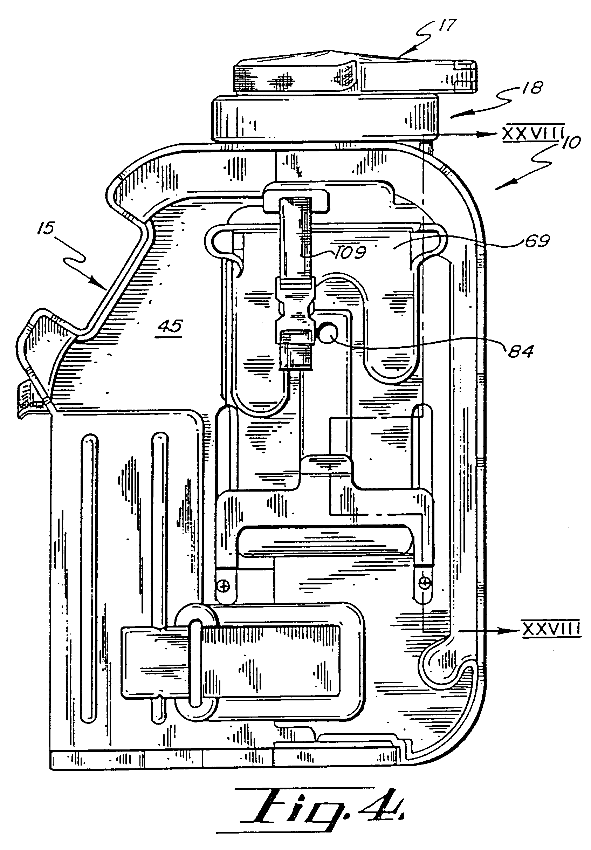

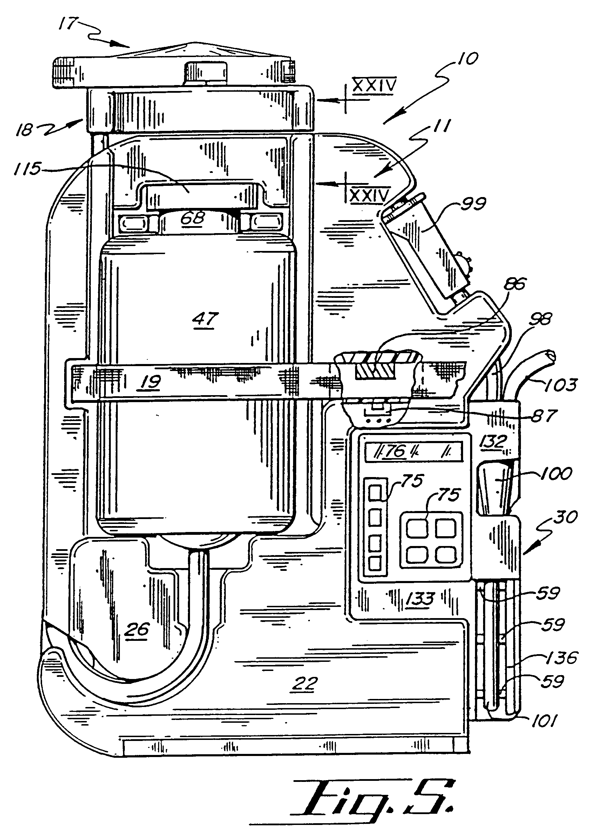

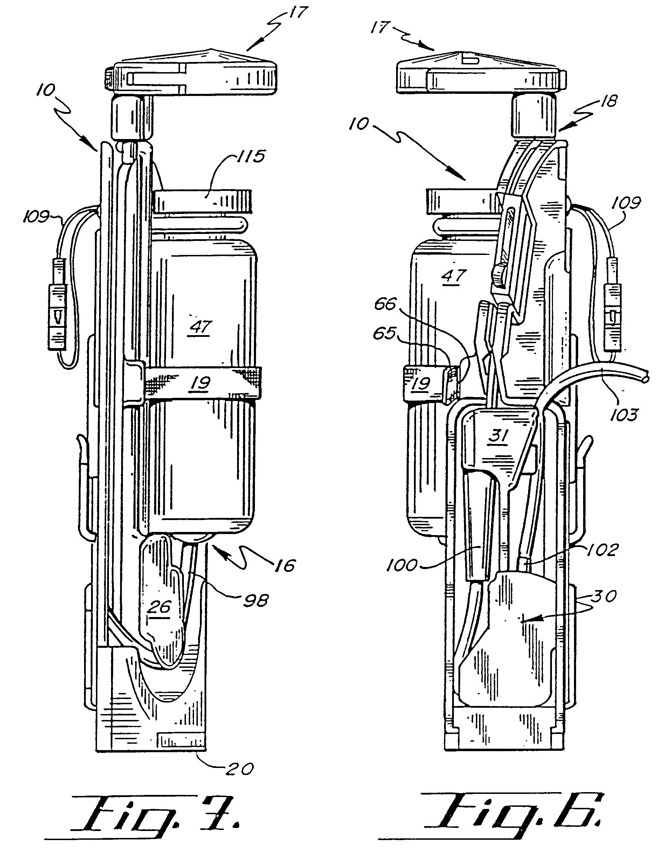

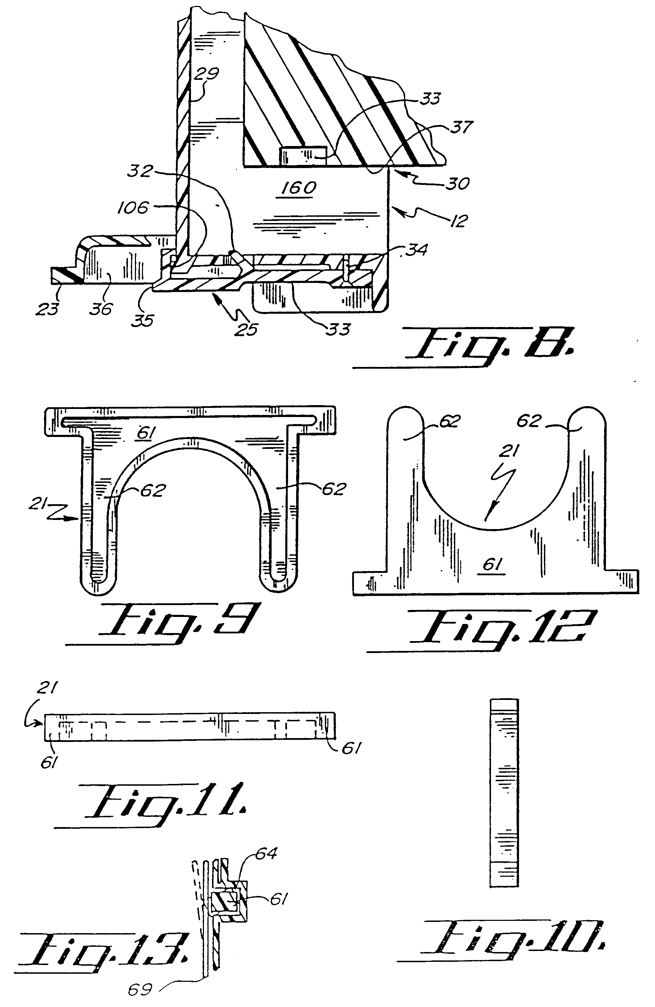

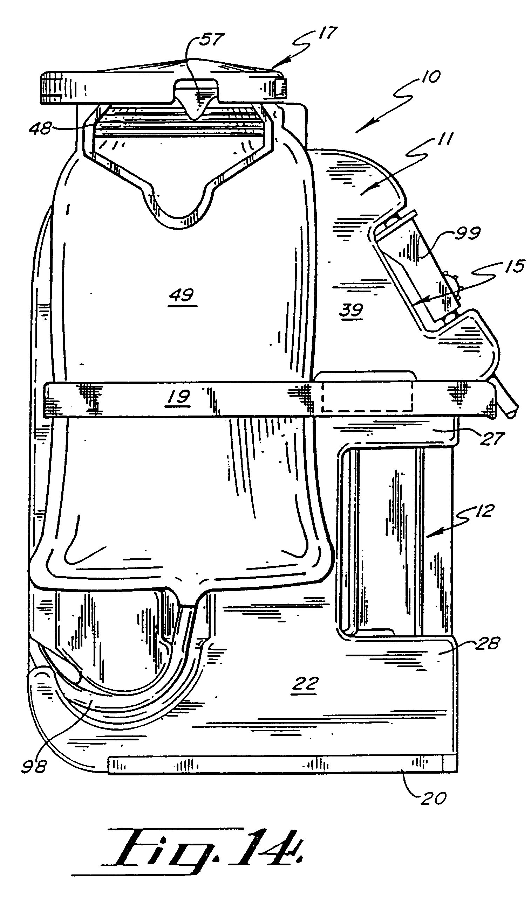

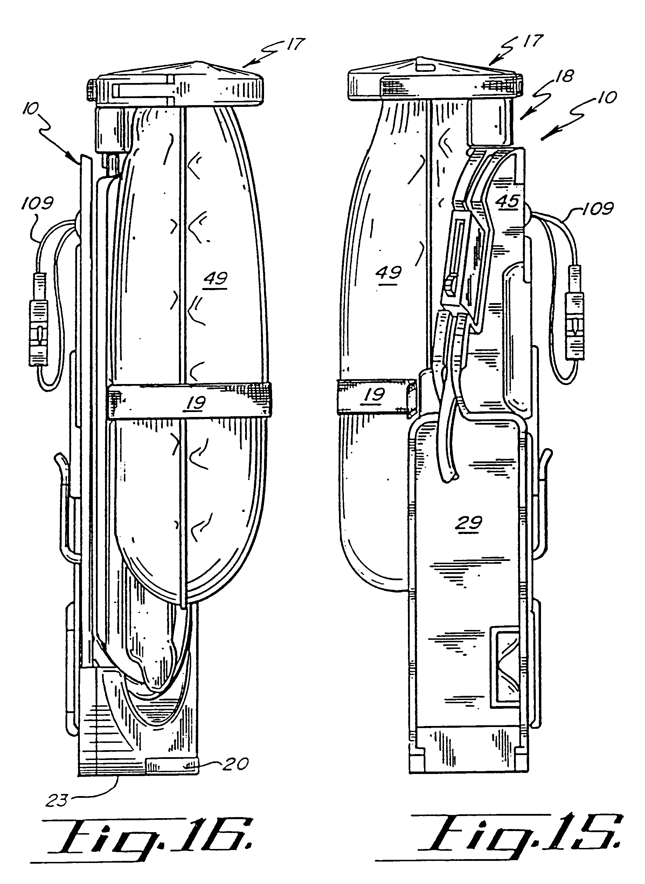

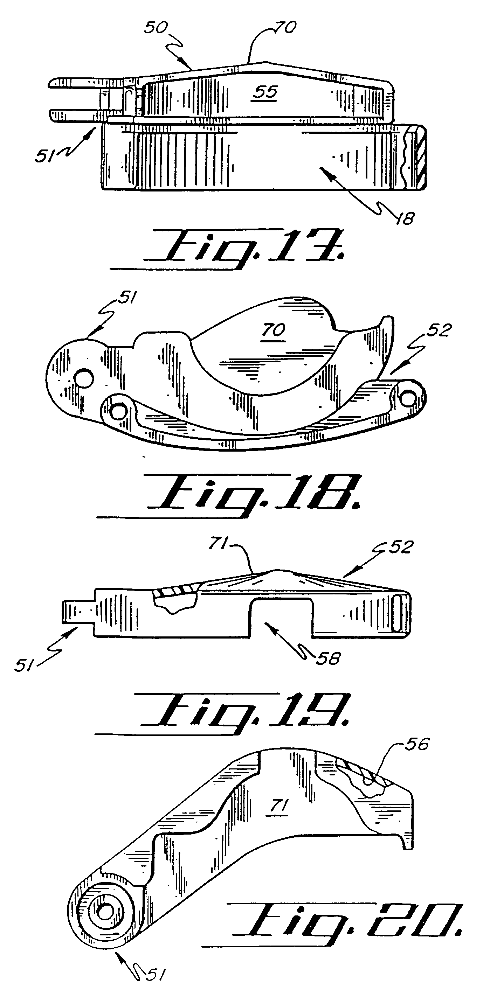

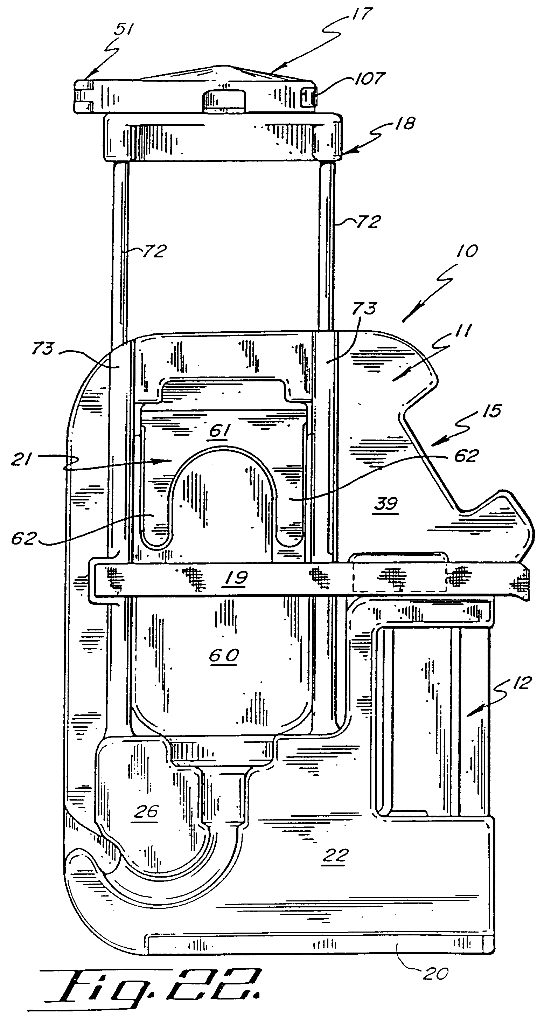

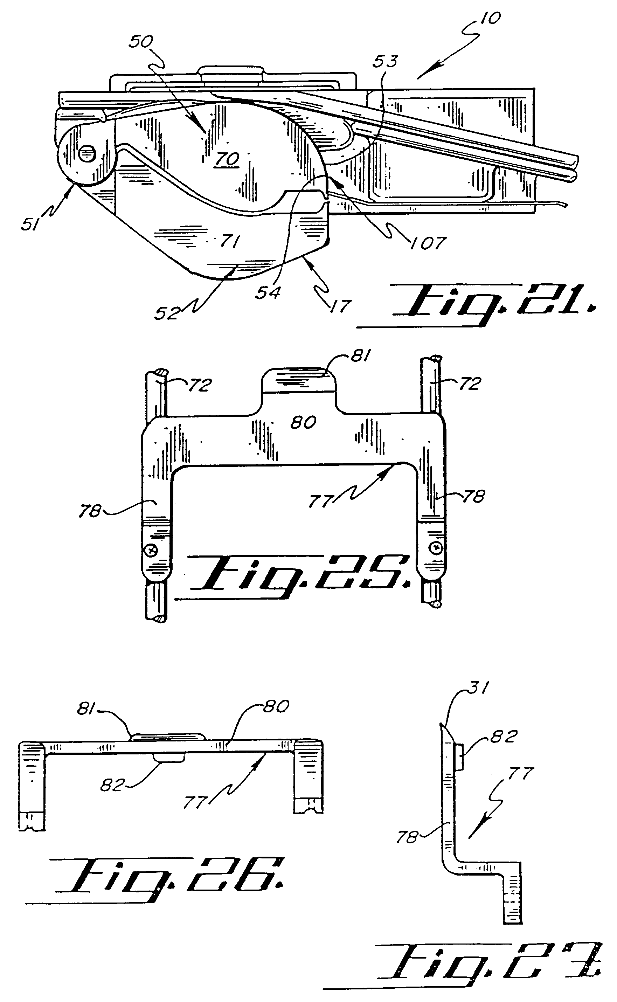

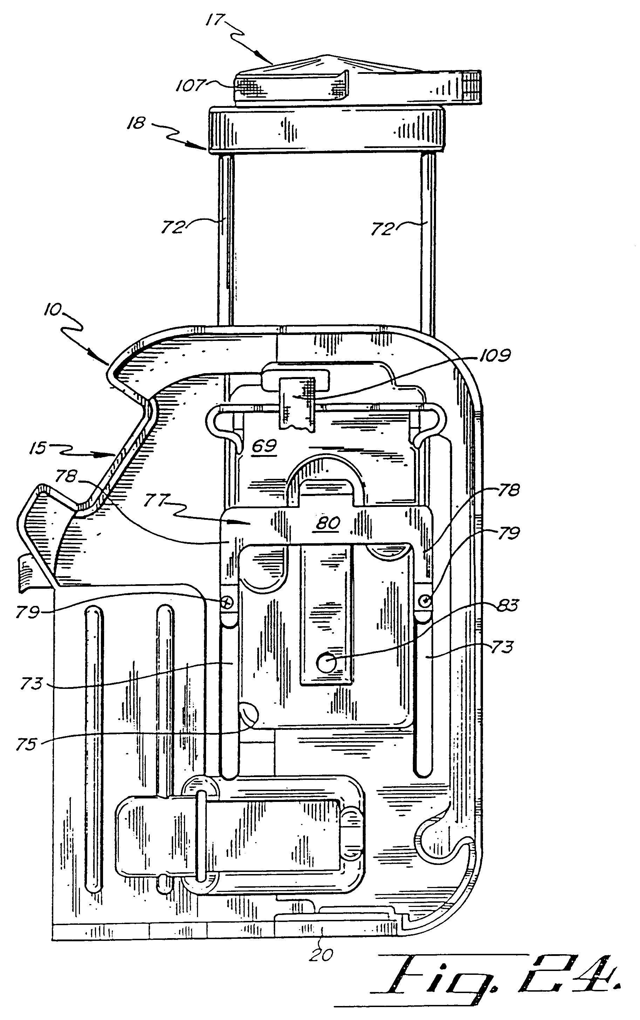

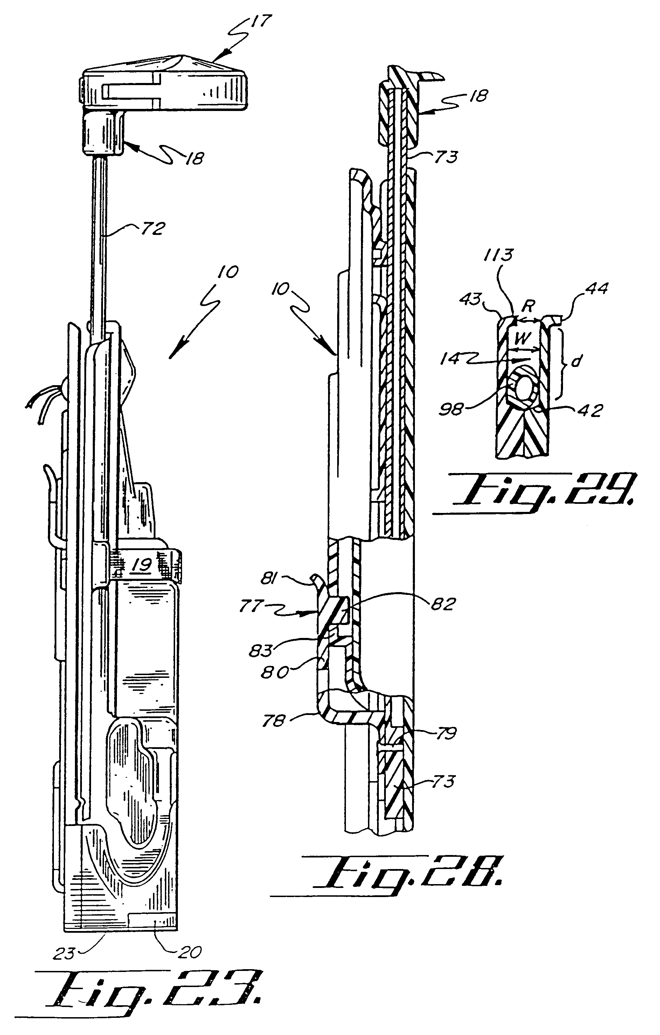

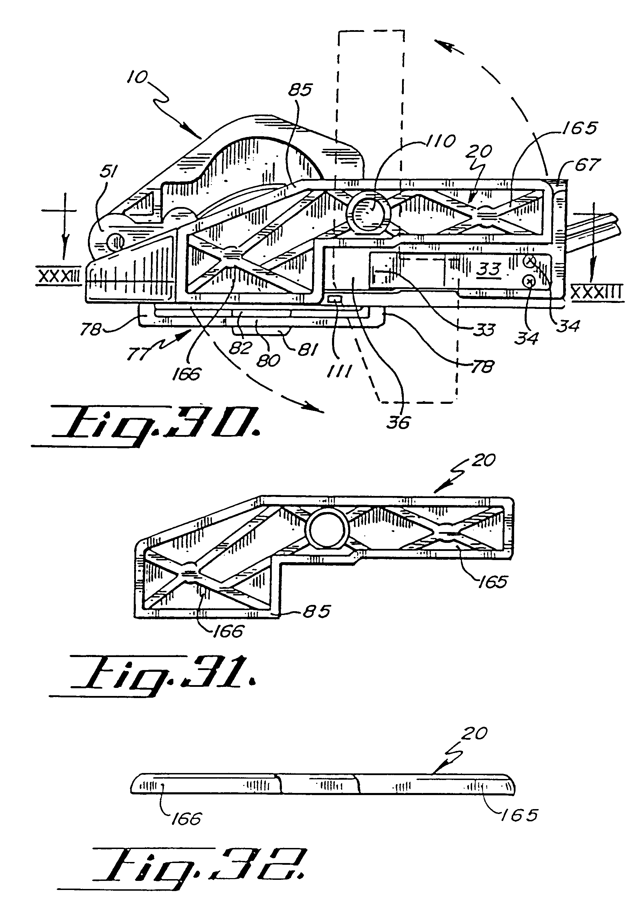

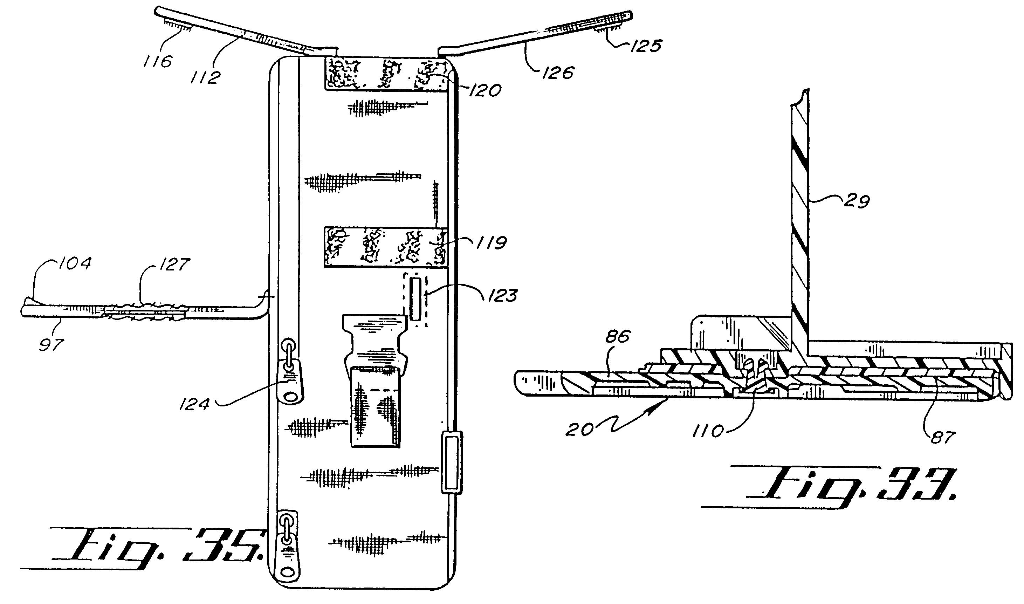

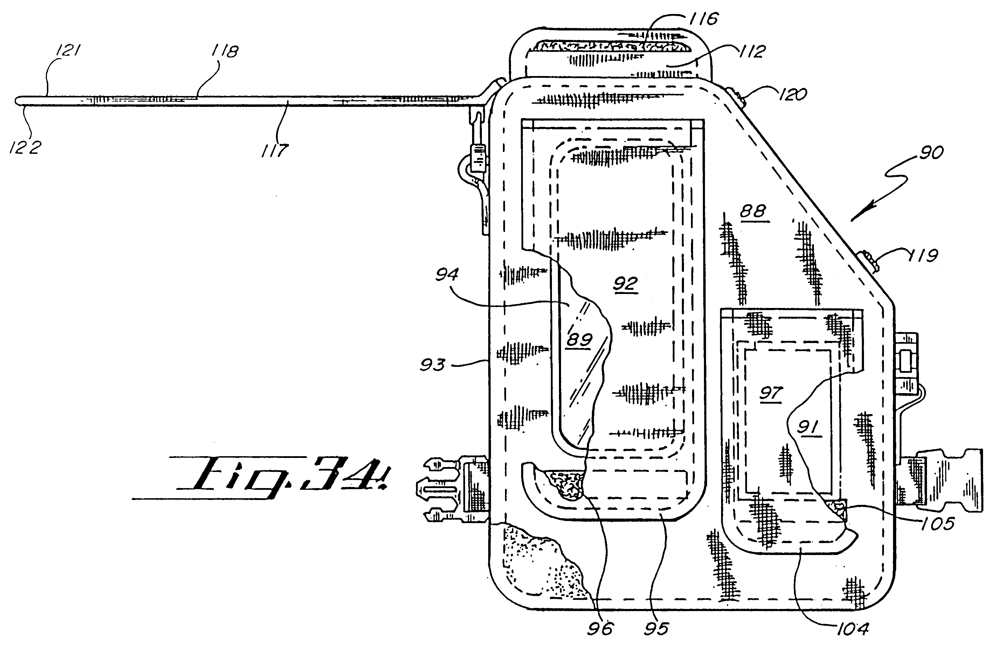

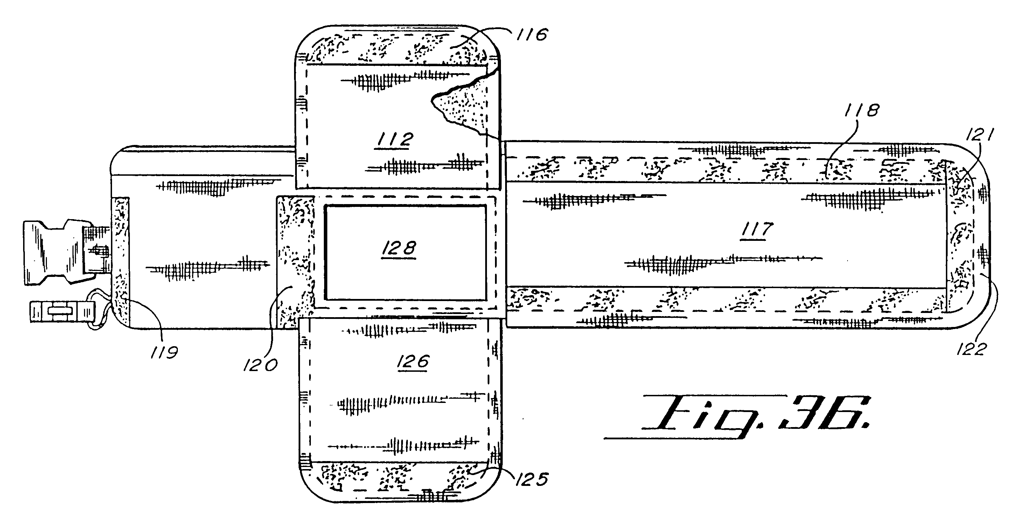

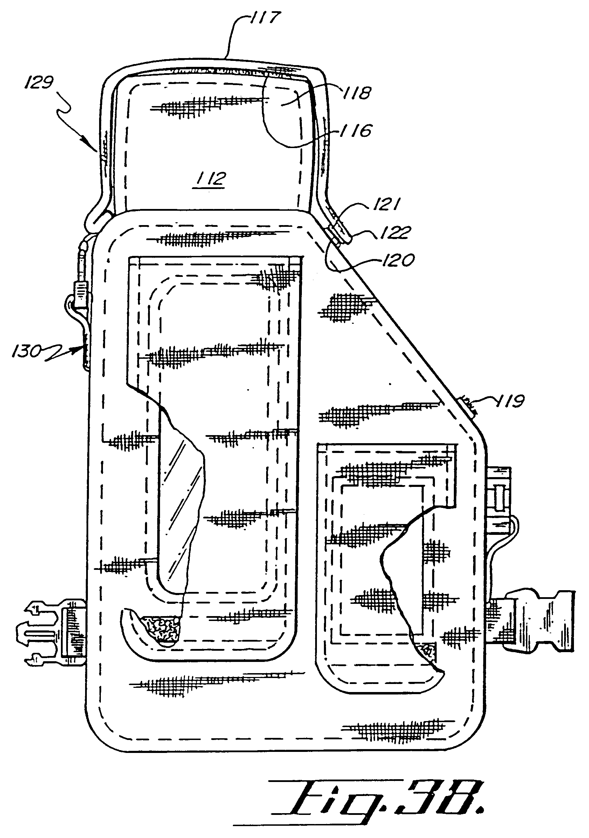

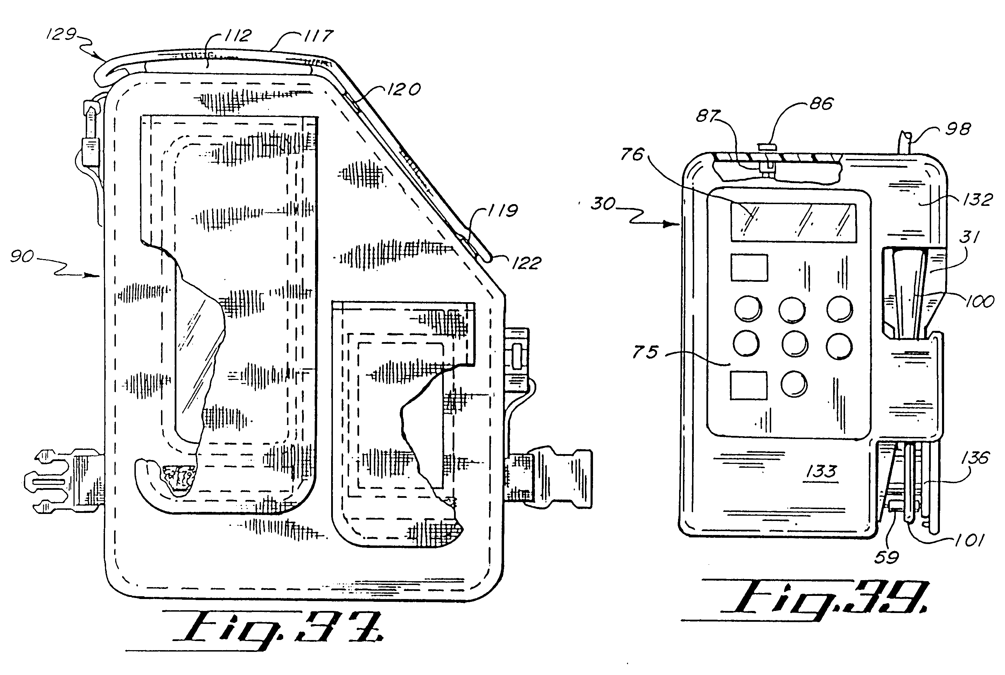

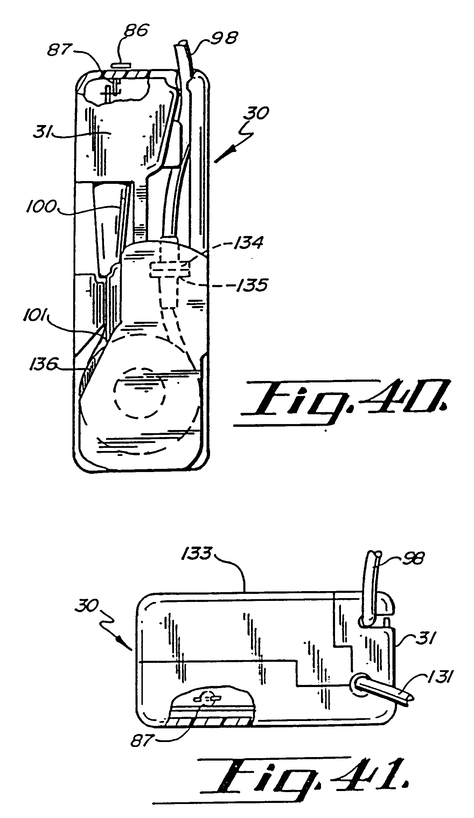

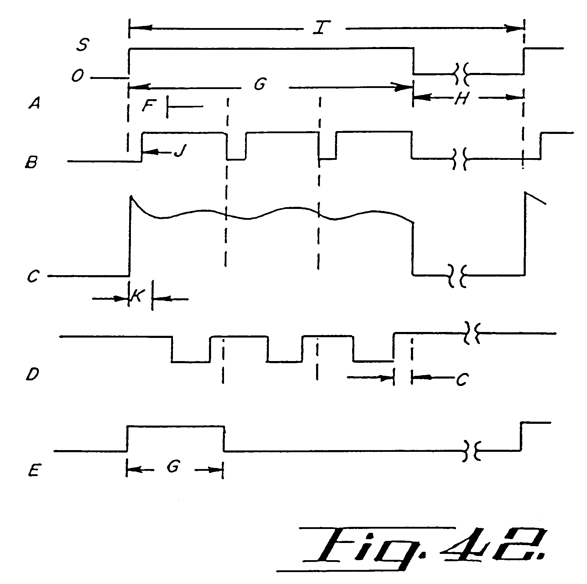

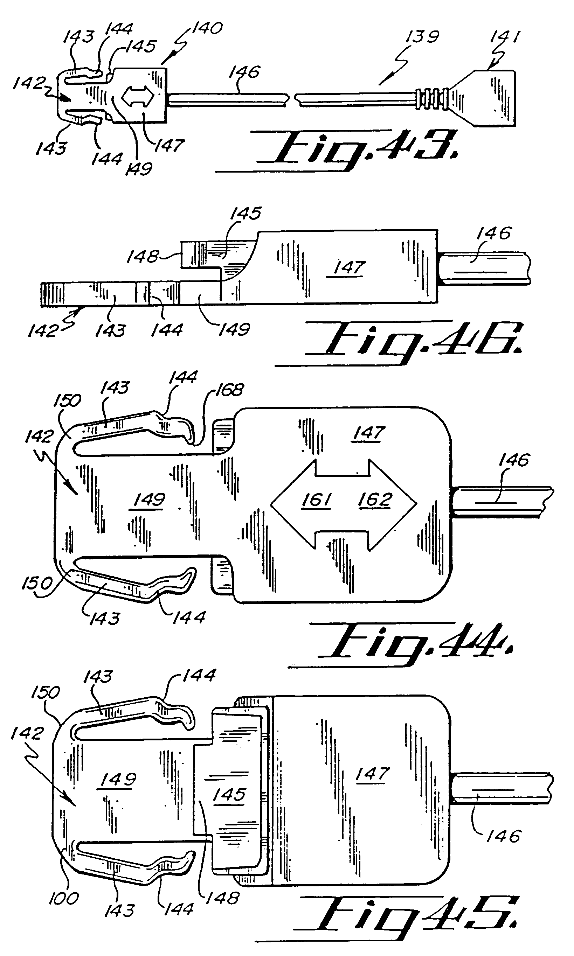

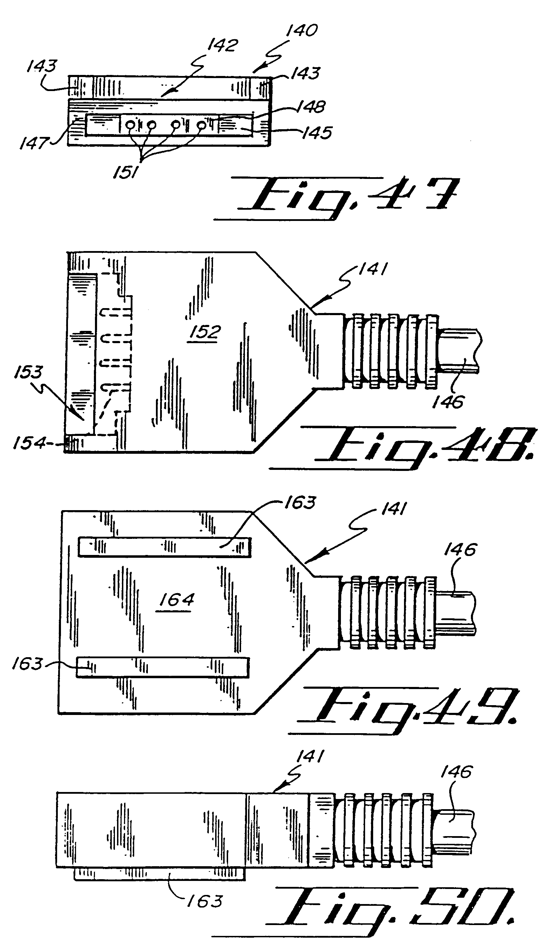

More specifically, as shown in Figures 1-4, the support device 10 includes a generally rectangular rigid body 11 which is preferably formed of a rigid plastic or other lightweight material such as wood, metal alloy, etc. Referring momentarily to Figures 5-8 in conjunction with Figures 1-4, the body 11 is adapted to receive and retain a fluid delivery set 16 and an infusion pump 30 of a fluid delivery system. Specifically, the body 11 forms a pump compartment 12 adapted to receive the standard infusion pump 30, a container compartment 13 adapted to partially receive a container 47 from the standard fluid set 16, a tube channel 14 adapted to receive the tube 98 of the standard fluid set 16, and a valve compartment 15 adapted to receive a pinch valve 99 located on the tube 98 of the fluid set 16.

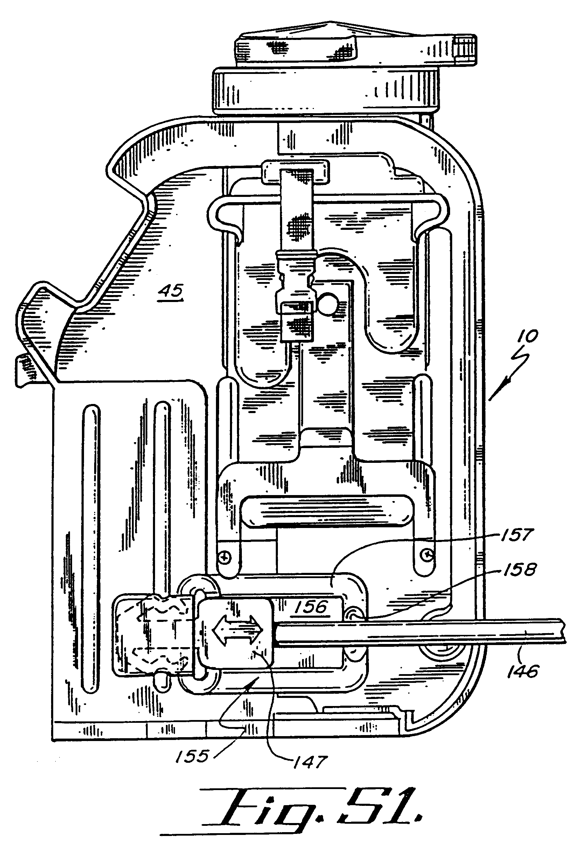

The support device 10 also includes a plurality of fastening elements which are adapted for use in securing the fluid delivery system to the rigid body 11 during use. These elements include a lid clamp 17 which is permanently affixed to a lid clamp extension 18, a securing strap 19, a saddle bracket 21 which is secured in a flush mount position in the bottom 60 of container compartment 13, and a pump locking mechanism 25 (best shown in Figures 2 and 8) formed as a part of the base 23 of the body 11.

The body 11 is also integrally formed with an elevated section 22 which forms a part of the pump compartment 12 and cooperates with a similarly elevated section 26 to form part of the tube path 40.

An extendable leg 20 may be located below elevated section 22 so as to be flush therewith when in its retracted position, and to be perpendicular therewith and parallel to base 23 when in its extended position.

The body 11 is preferably formed by a vacuum forming process well known in the prior art, which includes vacuum forming a front portion 39 separate and apart from a back portion 45, and then permanently interconnecting the portions to complete the formation of the rigid body 11 in a well known manner. Alternatively, a reaction injection or other injection molding technology may be used to form the body 11.

Turning now to a more detailed description of each main inventive feature of the support device 10, the pump compartment 12 is formed of a generally C-shaped cavity including upper and lower U-shaped channels 27 and 28, respectively, which are sized to allow the pump 30 to slide into the compartment 12 until it makes contact with the vertical abutment surface 29. The upper U-shaped channel 27 is formed contiguously with the raised surface 22 of the front portion 39 and the back portion 45, with the end of the tube channel 14 being located adjacent thereto and formed from the juncture of the front and back portions 39 and 45 as will be explained in more detail below. The lower U-shaped channel 28 is formed contiguously with the raised surface 22 of the front portion 39, the back portion 45, and the base 23.

As shown in Figure 5, the upper channel 27 of the pump compartment 12 preferably includes a sensed member 86 therein. The sensed member 86 is preferably positioned so that it will be located immediately adjacent to the top surface of the pump 30 when the pump 30 is received in the pump compartment 12 of the support device 11. A preferred form of the sensed member is a magnetic field source composed of 88% strontium ferrite and 12% #6 nylon having a magnetic strength of about 400 to 500 gauss at the surface edge.

The pump 30 includes a sensor member 87 positioned within its top inner surface. The sensing member 87 is preferably a three pin digital magnetoresistive sensor. This type of sensor member 87 has been found to be particularly advantageous because it has an omnipolar magnetic feature which allows it to be activated or released by either the north or south pole of the sensed member 86. Testing of the preferred forms of the sensed member 86 and sensing member 87 indicate that the respective members may be misaligned up to about 0.1 inch (.25 cm) without affecting the direction of the sensed member 86 by the sensor member 87. When the pump 30 senses the presence of sensed member 86, it automatically changes its mode of operation from non-ambulatory use to ambulatory use.

The pump 30 provides an intermittent motor operation, with a periodicity or cycle time of the intermittent operation being regulated to adjust to the desired rate of fluid delivery as disclosed more fully in U.S. Patent No. 4,884,013. The operation of the motor unit 62 is preferably cyclical and will be explained with respect to timing diagrams of Figure 42.

Graph A of Figure 42 illustrates the motor voltage of the enteral fluid delivery system. The motor voltage is turned on and operated for a time period G which is regulated by detecting the rotation of the rotor 136 (as shown in Figure 39), in the case of Graph A for one complete revolution. During one complete revolution, represented by motor voltage period G, all three rotor magnets pass the magnetic field detector which is located adjacent to the rotor 136 in the housing assembly 133 and are sensed thereby.

Curve B in Figure 42 illustrates the output signal from the rotor magnet sensing magnetic field detector which occurs during the cycle of operation indicted by motor voltage G. During an initial period of approximately 0.45 seconds designated F in Figure 42, the operation of the rotor sensing magnetic field detector is inhibited by software in the pump 30 microcomputer so that the initial on period J of the magnetic field detector is not responded to by the control program. Thereafter, during one complete revolution of the rotor 136, the signal from the detector goes to zero as each magnet is encountered by the detector. Upon detection of the third magnet, at the end of period G, the motor voltage is turned off.

In accordance with the preferred embodiment of the present invention, the pump 30 repeats the cyclical operation a time period I (the cycle time) after initiation of the first operation. The time period H during which there is provided no motor voltage is permitted to be variable, since it depends on the actual time taken for rotation of the rotor 136 and the selected interval I. The interval I is selected according to the rate of fluid delivery which is set by the operator with the preferred form of the infusion pump 30.

The pump 30 operates under the control of a microcomputer which is provided with first and second control programs. The pump 30 controls the operation and rotation of the rotor 136. A programmable interval timer is provided for operating and initiating the microcomputer. A clock, preferably operating at approximately 4 Mhz, provides clock pulses to the pump 30. The various controls of the unit are provided as input signals which ground various input terminals of the microcomputer to thereby signal the operators input instructions.

As shown in Figures 39-41, the LED display 76 is driven by the microcomputer. Additional inputs to the microcomputer are provided by the three magnetic field sensors which detect the magnetized mounting member 134; the magnets on the rotor 136 and the sensed member 86 on the support device 10. Likewise, a drop detector is connected to provide input signals to the microcomputer. An AC power rectifier is provided for AC operation and battery charging. Portable DC operation is available using a NiCAD battery. The AC circuit is arranged to charge the DC battery when the unit is connected to AC power. A low battery and dead battery detector circuit is provided to signal the microcomputer that the battery needs recharging.

The microcomputer provides an output motor signal which is coupled by transistor to a plurality of switching transistors. The transistor turns on the power supply to the motor voltage regulator when the pump 30 is to be operated and the transistor short circuits the pump 30 to lock it into position when the motor signal is no longer present. The switching transistor which is provided with a power signal by the transistor, operates to supply current to the motor and the other electronic systems of the pump 30 by the voltage regulator when the power is turned on. The pump 30 is provided with a fail safe circuit which creates a short circuit when the pump 30 is operated for an excessive period of time as disclosed more fully in U.S. Patent No. 4,833,379. The short circuit causes a fuse to open, thereby disabling the pump 30 when continuous motor operation occurs, to avoid pumping excess fluid to a patient.

The foregoing is illustrative of the preferred form of the pump 30. When the pump 30 is used in a non-ambulatory setting, without being attached to the support device 10, the sensor member 87 is not activated and there is no additional input provided to the microcomputer. In this situation, the cycle time or period I (Figure 42) may vary from about 450 seconds for each rotor magnet sensed to provide a fluid rate of 1 ml/hr, to about 6.75 seconds for the sensing of three rotor magnets to provide a fluid rate of 200 ml/hr. Other illustrative cycle times are about 9 seconds for the sensing of a single rotor magnet to provide a fluid rate of 50 ml/hr, and about 13.5 seconds for the sensing of three rotor magnets to provide a fluid rate of 100 ml/hr. As stated previously, in this situation, the pump 30 is operating in the manner disclosed in U.S. Patent No. 4,884,013. This is because the first control program in the microcomputer is selected based on the fluid pressure created in the pump tube 101 when the container 47 is extended above the infusion pump 30 on an IV pole (not shown).

When the infusion pump 30 is used with the support device 10 as shown in Figures 5-8, the fluid pressure in the pump tube 101 is significantly lower. Therefore, the pump tube 101 will contain less fluid between the rollers 59 of the rotor 136 and less fluid will be delivered to the patient unless the operation of the pump 30 is increased to increase the rotation of the rotor 136 accordingly. As best shown in Figure 5, when the pump 30 is used with the support device 10, the bottom of the container 47 is positioned only slightly above the rotor 136 of the pump 30 and therefore, the fluid pressure within the pump tube 101 is significantly reduced as compared to when the container 47 is on an IV pole.

The sensor member 87 of the present invention provides an indirect and on/off indication that the pump 30 is being used in an ambulatory manner. Therefore, when the pump 30 is placed in the support device 10 as shown in Figures 5-7, the magnet or sensed member 86 on the support device 10 is sensed by the magnetic field detector or sensor member 87 in the pump 30. When the sensor member 87 is activated by the sensed member 86, the sensor member 87 provides an input signal to the microcomputer to switch the microcomputer from the first control program to the modified or second control program. In the preferred form of the present invention, the second control program may be either a subprogram of the first control program or a completely separate control program. In either form, the second control program decreases the cycle time (Period I in Figure 42) to account for the reduced fluid pressure within the pump tube 101. With the present invention, the cycle time is decreased from about 5% to about 10% and more preferably 8.85% so that for a 1 ml/hr. fluid delivery rate, a single rotor magnet is sensed approximately every 410 seconds and for a 200 ml/hr. fluid delivery rate, three rotor magnets are sensed approximately every 6.16 seconds. The other cycle times are similarly reduced such that for a fluid delivery rate of 400 ml/hr., three rotor magnets are sensed approximately every 3.08 seconds when the sensor member 87 is activated as compared to approximately every 3.38 seconds when the sensor member 87 is not activated.

In addition to the above-described preferred form of the present invention, it is anticipated that the sensor member 87 may be used on nearly any medical fluid infusion pump 30 and the sensed member 86 may be mounted directly on a portion of the fluid delivery set 16. Additionally, the sensor member 87 may be modified to provide a continuous input signal to the microprocessor wherein the signal indicates the distance between the container 47 and the rotor 136 of the pump 30. This may be accomplished with various sensors such as an infrared sensor or a fluid pressure sensor, or it may be part of the input information required from the user prior to operation of the pump 30. The control program may then be modified to adjust the cycle time of the rotor 136 to provide an accurate infusion rate based on the known fluid pressures created in the pump tube 101 at various distances above the pump 30 or rotor 136.

As best shown in Figures 2 and 8, the pump locking mechanism 25 includes an upwardly extending locking pin 32 which protrudes into the pump compartment 12. The pin 32 is integrally formed with a lever arm 33 which in turn is connected to the base 23. It is intended that the arm 33 be somewhat flexible and may be a separate component affixed to the base 23 (as shown by screw 34) or may be integrally molded therewith. The opposite end of the lever arm 33, adjacent lock pin 32, includes a release tab 35 which can be accessed through base opening 36 by a users finger in order to move the lock pin 32 into and out of locking position within compartment 12. A stop member 106 is positioned to engage with release tab 35 when the lock pin 32 is moved out of locking position in order to prevent over flexion of the lever arm 33.

As best shown in Figure 8, the bottom surface 37 of the pump 30 preferably includes a detent 38 which is sized and positioned so as to allow lock pin 32 to snap thereinto when the pump 30 is properly inserted within the pump compartment 12. Positioning of the pump 30 within pump compartment 12 is accomplished by sliding the pump 30 into the upper and lower U-shaped channels 27 and 28. While the pump 30 is moving into the pump compartment 12, the bottom surface 37 thereof initially pushes lock pin 32 in a downward direction until the detent 38 becomes positioned thereover (as the pump 30 abuts vertical wall 29), at which point the locking pin 32 snaps into position into the detent 38. The locking pin 32 then holds the pump within the pump compartment 12 until such time as the user pulls release tab 35 downwardly to withdraw lock pin 32 from the detent 38 and slides the pump 30 out of the pump compartment 12.

Turning now to Figures 5-7, the support device 10 of the present invention is shown to be adapted to receive and secure a rigid blow molded plastic bottle type container 47 commonly used with a standard fluid delivery set 16. As can be seen in Figure 1, the container compartment 13 is recessed below front surface 39 of the body 11, and shaped to receive a portion of the bottle 47 in a preferred position relative to the pump 30.

The saddle bracket 21 is located within compartment 13 and flush mounted with the bottom 60 thereof so as to be out of the way when not in use. The bracket 21 includes a cross bar 61 and a pair of bracket arms 62 which extend perpendicularly therefrom and which are spaced apart from each other a distance slightly greater than the diameter of the neck 68 of a standard feeding bottle (such as the rigid plastic bottle 47). The bracket 21 can remain flush mounted within bottom 60 of the container compartment 13 when not in use, or can be rotated 90 degrees to cause the bracket arm 62 to extend perpendicularly from bottom 60 of the container compartment 13 and fit around the neck 68 of the bottle 47 to aid in maintaining it in its proper position during use. The saddle bracket 21 can be used in a similar manner to maintain the neck of a burette or other type of fluid container during use.

The saddle bracket 21 rests within a cavity 63 in the bottom 60 of the container compartment 13. The cross bar 61 extends beyond the bracket arms 62 to pass into bracket arm mounting holes 64. In use, the saddle bracket 21 is lifted into its upright position by pulling bracket arm 62 upwardly from the cavity 63. This is most easily accomplished by inserting a finger into the cavity extension 108 and leveraging the arm 62 slightly out of the cavity 63. Bracket arm 62 can then be rotated until the arm 62 is in a perpendicular position by gripping the arm 62 from the container compartment 13 and rotating upwardly.

As best shown in Figure 13, the cross bar 61 and the bracket arm mounting hole 64 are preferably formed into square cross-sectional shapes with a deflectable wall 69 which allows a slight resilient deformation of the mounting hole 64 as the cross bar is rotated therein. Such a design causes the arms 62 of the bracket 21 to be biased into a flush position with bottom 60 of the container compartment 13 until they are rotated approximately forty-five degrees at which point the cross bar 61 is biased to rotate to a perpendicular position where it is again properly oriented within mounting hole 64. Such a mounting design is herein referred to as a "snap up" and/or "snap down" mounting.

If desired for additional support of a container placed in container compartment 13, a strap 19 can be located on the front surface 39 of the body 11. The strap 19 is preferably positioned adjacent the container compartment 13 and of a sufficient length to cross over a container placed in container compartment 13 and be attached to the upper U-shaped channel 27 of the pump compartment 12. The attachment may be made in any convenient manner such as by hook and pile fasteners 65 and 66, respectively.

As best seen in Figures 14-16, the device 10 is also adaptable to receive a fluid set which includes a soft flexible fluid bag 49. The compartment 13 operates in conjunction with lid clamp 17 to hold the bag 49 in place. The lid clamp 17 includes a inner jaw 50 permanently attached to a lid clamp extension 18, and attached through hinge 51 to an outer jaw 52. A fastener, such as strap 107 including the pile portion 53 of a hook and pile type fastener, is attached to jaw 52, with the hook portion 54 of the fastener attached to jaw 50. The strap 107 allows the clamp 17 to be securely fixed in a closed position when the lid 48 of the soft bag 49 is located therein. When in the closed position, the jaws 50 and 52 of the clamp 17 form a circular opening which hold the mouth and lid 48 of the bag 49 in place on the support device 10.

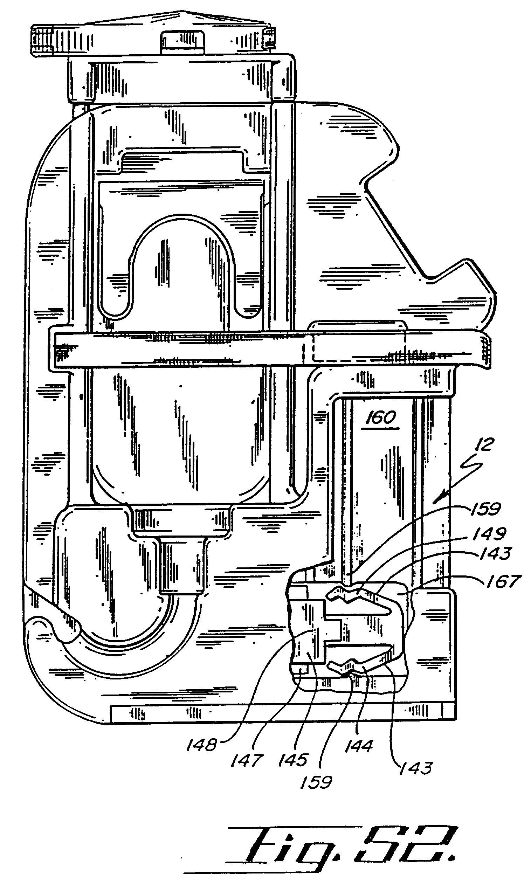

As shown in Figures 17-18 and Figures 19-20, the circular opening of the inner clamp jaw 50 forms an inner lip channel 55, and similarly, the outer clamp jaw 52 forms an outer lip channel 56 which receive the circumferential edges of mouth and lid 48 of the bag 49. Also, (see Figure 14) since the lid 48 generally includes an opening tab 57 thereon, the outer clamp jaw 52 is formed with a tab opening 58 through which the tab 57 can extend when the clamp 17 is closed about the lid 48.

As shown in Figure 21, the jaw members 50 and 52 include convex plate extensions 70 and 71 respectively which together form a generally dome-shaped surface which can effectively accommodate a bulging shape taken on by the lid 48 in the event of sudden pressurization of the bag 49 which could occur if dropped.

The lid clamp 17 operates to secure the lid 48 of bag 49 in its proper position and allow the bag 49 to be properly located within container compartment 13. Also, and more importantly, the lid clamp 17 operates to prevent the sudden application of an external pressure from inadvertently bursting the lid 48 open during use such as may occur if the support device 10 is inadvertently dropped.

The lid clamp 17 can be positioned above container compartment 13 a sufficient distance to allow the accommodation of the desired size of bag 49. For example, as shown in Figures 14-16, the lid clamp extension 18 may be located directly adjacent the body 11 to allow the container compartment 13 to accept and properly position a bag 49 of standard 600 ml. volume. Alternatively, as shown in Figures 22-24, the lid clamp extension 18 can be moved to a predetermined position which is a sufficient distance from the body 11 to allow room in container compartment 13 to accept a bag 49 of a standard 1000 ml. volume.

The lid clamp extension 18 is mounted for movement relative to the body 11 by means of extension rods 72. The rods 72 are mounted in the front portion 39 of the body 11 through the tubular channels 73.

As best shown in Figure 24, slots 74 extend through back portion 45 of the body 11 to expose the tubular channels 73. A U-shaped extension locking member 77, having arms 78 is attached to the bottom of each extension rod 72 such as by means of screws 79 or in any other well known manner.

As best shown in Figure 24 and Figures 25-27, the U-shaped extension locking member 77 includes a bail portion 80 which extends between the arms 78 and includes a release tab 81 and locking pin 82 formed at a generally central location thereon.

As best shown in Figure 28, the locking member 77 is designed to allow locking pin 82 to be positioned within opening 83 in the back portion 45 of the body 11 in order to lock the lid clamp 17 in position adjacent the body 11 (see Figure 4). When it is desired to move the lid clamp 17 to an extended position away from the body 11, the release tab 81 is lifted away from the back portion 45 of the body 11 to disengage locking pin 82 from opening 83. The extension locking member 77 is then pushed in the upward direction until locking pin 82 can engage opening 84. As the extension locking member 77 is moved in an upward direction, the arms 78 thereof attached to the extension rod 72 force the extension rod 72 to slide upwardly in slots 74 and force the extension rods 72 to slide in the upper direction in the tubular channel 73. Once the locking pin 82 is engaged in opening 84, the lid clamp 17 is properly located in its extended position (see Figure 24).

As best shown in Figures 1-3, the tube channel 14 of the support device 10 extends around approximately two thirds of the circumference of the body 11. The tube channel 14 is generally U-shaped in cross-section and includes a base 42, a front wall 43, and a back wall 44.

The tube channel 14 is essentially a channel between the front and back portions 39 and 45, respectively, of the body 11, and extends from entrance opening 41 across the top and partially down the opposite side of body 11 to exit opening 46. Slightly above exit opening 46, the channel 14 is interrupted by a pinch clamp compartment 15 which is sized to receive the standard type pinch clamp 99 commonly attached to the tubing 98 of a fluid delivery set 16. The pinch clamp compartment 15 is formed by a cut out section of the body 11, and is sufficiently large to allow the pinch clamp 99 (see Figure 5) to rest therein when the tubing 98 is located in the tube channel 14.

As can be seen in Figure 29, the channel 14 is designed to allow accommodation of the tubing of the fluid set 16 even though slight variations in length thereof may occur. This is because the channel 14 is of sufficient depth (d) to allow some "snaking" of the tube within the channel 14 if necessary to accommodate its entire length.

Further, the channel 14 is also designed to retain the tubing therein once place, even though some "snaking" may occur. Specifically, the channel 14 is generally of a width (w) which is slightly larger than the diameter of the tube 98. However, at the top of the channel 14, the width (R) is restricted to a dimension less than the diameter of the tube 98. The restriction is in the form of a lip 113 which ensures that the tubing 98 stays within the channel 14. Without the pressure of the lip 113, the tube 98 would have the mechanical inclination to bow outwardly and at least partially escape the channel 14 at various locations around the body 11.

Due to the presence of the lip 113, it is advantageous to form a tapper 114 at the inlet opening 41 of the channel 14 for ease of beginning the insertion of the tube 98 into the channel 14.

Because of the restricted width (R) of the channel 14 at the lip 113, the tube 98 becomes resiliently deformed into an oval cross-sectional shape while passing into the channel 14. Once the tube 98 is forced entirely within the channel 14, it will return to its circular cross-sectional shape and will thereafter be retained within the channel 14 until forcibly withdrawn therefrom.

Located below container compartment 13 are the elevated sections 22 and 26 of the body 11 which are oriented to form a tube path 40 for passing a tube from a container placed in container compartment 13 to the entrance 41 of the tube channel 14.

As best illustrated in Figures 1 and 2, the forward sloping section 24 of the body 11 causes the exit opening 46 of the tube channel 14 to be positioned somewhat centrally over the pump compartment 12. This is advantageous in that it allows the tubing 98 of the fluid delivery set to pass from channel 14 at exit opening 46 in the proper position for reception into the hinged pump arm 31 of the pump 30.

The support device 10 is adapted to be used with the pump 30 and fluid delivery set 16 in a variety of environments. For example, body 11 of the support device 10 may be mounted to a standard infusion pole for use with bed ridden or ambulatory patients by means of mounting strap 109.

Alternatively, the device 10 may be placed into a carrying case 90 and strapped to the patient's back for ambulatory use. As best shown in Figure 34, the case 90 may be utilized to store the device 10, along with the pump 30 and fluid delivery set 16 attached thereto, in order to allow complete and convenient ambulatory use thereof.

The case 90 is formed generally to conform to the exterior shape of the support device 10 and includes semi-rigid foam lined walls 93. The front 88 of the case 90 includes a visual access opening 94, covered with a clear plastic panel 89, which allows visual access to the container 47 when mounted to the support device 10 for use.

Front 88 of the case 90 also includes a control panel opening 91 which allows visual and physical access to the control panel 75 and display 76 of the pump 30.

The opening 94 is covered by a flap 92 which is sized so as to cover the entire opening 94 in a protective manner. The flap 92 can include an opening tab 95 and a fastening means such as hook and pile fastener 96.

Control panel opening 91 also includes a flap 97 sized to completely cover the opening 91 to protect the pump 30. Flap 97 may also include an opening tab 104 and hook and pile type fastener 105. Further, as best shown in Figure 35, flap 97 may also include a semi-rigid protection panel 127 which will supply added protection against accidental control panel 75 activation, or damage to the pump 30 due to an inadvertent blow to the case 90. The case 90 can include any number of carrying straps for allowing the case to be carried on the shoulder, back, or around the waist of the patient while ambulatory.

As best shown in Figure 35, the case also includes a tube outlet opening 123 to allow tubing 103 (see Figure 5) exiting the pump 30, to pass outside of the case 90 to be attached to the patient.

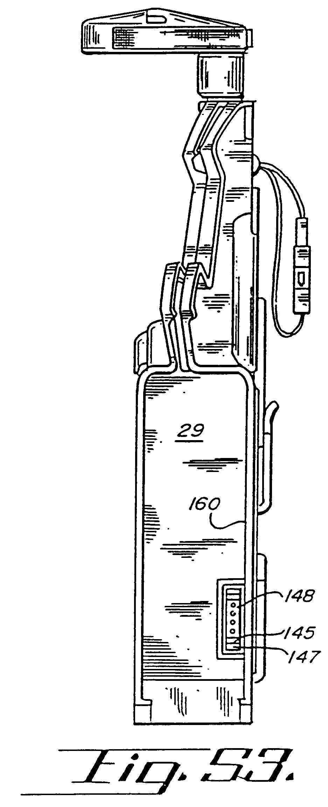

As shown in Figure 36, the top of case 90 is formed with an opening 128 therethrough which is adapted to allow the lid clamp extension 18 to extend therethrough when in its extended position holding an enlarged bag 49 of a standard fluid set 16. Adjacent the opening 128, in diametrically opposed positions, are front side flap 112 and back side flap 126. Also adjacent opening 128, in a position aligned with the side surfaces of the bag 90 is a central flap 117. Flaps 112 and 126 are of a length which is substantially equal to the extension length of the lid clamp extension 18 of the support device 10. The central flap 117 is of a length which approximates the distance around the perimeter of the front and back side flaps 112 and 126 respectively.

As best shown in Figures 36 and 37, when there is no need for extending the pack 90, e.g. when the lid clamp extension 18 is in the non-extended position relative to rigid body 11, and a small bag 49 (or container 47) is located in the support device 10, the front and back side flaps 112 and 126 are folded over the opening 128 and the central flap 117 is then extended over the folded front and back side flaps 112 and 126 until the end 122 of the central flap 117 extends a sufficient distance around the side of the case 90 to allow end fastener 121 to attach with the closed position fastener 119 on the case 90.

As best shown in Figure 38, when it is necessary to extend the case 90 to accommodate an extended lid clamp extension 18 and large bag 49, e.g. when lid clamp extension 18 is in its extended position relative to rigid body 11 for purposes of receiving a large bag 49, the front and back side flaps 112 and 126 respectively are extended vertically in the manner shown in Figure 38, and the front and back side flap fasteners 116 and 125 respectively are folded inwardly so as to rest on top of the lid clamp extension 18. The central flap 117 is then extended over the lid clamp extension 18 and the central flap fastener 118 engages with the front and back flap fasteners 116 and 125 respectively. Extension of central flap 117 is continued until the end fastener 121 thereof engages with the open position fastener 120 located on the side of the case 90. In this position, the support device 10 and the bag 49 are completely enclosed within the extension 129 of the case 90.

Alternatively, the device 10 may be placed on its base 23 on a level surface such as a table or the like without the need for any other mounting aid. In such an instance, the support leg 20 located in the base 23 of the device 10 may be used to add stability to the base 23 during use.

As shown in Figure 30, the leg 20 can be rotated to an open position parallel with the front and back portions 39 and 45 of the body 11 and flush with base 23 to prevent inadvertent tipping of the device in the forward or backward direction.

As shown in Figures 30 and 33, the leg 20 is mounted on the bottom of the support device 10 in recess 67 in such a manner to form a substantial portion of the base 23 thereof. The leg 20 is mounted for rotation about pivot pin 110. As shown in dash lines in Figure 30, the leg 20 can rotate approximately 90 degrees until locking edge 85 thereof passes completely beyond the locking surface 111 in which position the leg 20 is maintained due to the frictional contact between the frictional locking surface 111 and the edge portion 85. In the extended position as shown in dash lines in Figure 30, the leg 20 includes a forward extension 165 and a backward extension 166 which are substantially perpendicular to both the height and width of the body 11, and parallel and flush with the remainder of the base 23.

As best shown in Figures 31-32, the leg member 20 is a generally elongated flat member which is shaped to fit completely within the perimeter dimensions of the base 23 of the support device 10 when in its retracted position.

As best shown in Figure 5, the fluid delivery set 16 includes a container such as the plastic bottle 47, with a standard length of tubing 98 extending from the bottom thereof. The tubing 98 includes a pinch clamp 99 thereon and a drip chamber 100 attached at its distal end. The drip chamber 100 is also attached to an extensible, relatively thin-walled pump roller tube 101 which is especially adapted for use with the pump 30. An extension ring 102 is attached to pump tubing 101 and functions to insure that the pump tubing 101 is properly stretched over the rollers 59 of the pump 30 when in use. Beyond extension ring 102 is an infusion tube 103 which is intended to be attached to the patient.

It is to be understood that although the present invention is described for use in conjunction with the specific fluid delivery sets 16 and a specific pump 30, any well known type fluid delivery set or infusion pump may be used with or adapted for use with the support device 10 of the present invention and remain within the intended scope and meaning of the present disclosure. Similarly, obvious adaptions to the support device 10 necessary to accommodate other well known type delivery sets and pumps are intended to fall within the scope of the present invention.

A preferred method of attachment of the fluid delivery set 16, including the rigid bottle 47, and the pump 30 to the support device 10 of the present invention is described as follows. As shown in Figures 5-7, the pump 30 is inserted into pump compartment 12 until it is locked in position by pump locking mechanism 25. The saddle bracket 21 is lifted to its "pop-up" position and the bottle 47 is inserted into the container compartment 13 until tubing 98 thereof can extend into the tube path 40. In this position, the bracket 21 secures the neck 68 of the bottle 47 against lateral movement. The strap 19 is then secured over the bottle 47 to prevent its escape from the compartment 13. Next, the tube 98 is grasped and forced into entrance 41 of tube channel 14 and drawn the entire length of channel 14 until pinch clamp 99 is reached.

Pinch clamp 99 is then adjusted along tubing 98 until it is oriented properly to be received in pinch clamp compartment 15. Tubing 98 is then extended through the remainder of tubing channel 14 and allowed to extend beyond exit 46.

Once the tube 98 is properly placed, the drip chamber 100 is inserted adjacent arm housing 132 into the opened arm 31 (not shown in the open position) of the pump 30 and pump tubing 101 is passed around the pump roller 59 until the retention ring 102 including the magnetized mounting member 134, is properly positioned in a slot 135 within the arm 31 in such a manner as will cause the tube 101 to be stretched over the roller 59 of the pump 30 when the arm 31 is moved to its closed position. The pump arm 31 is then rotated into its closed and operating position and the infusion tube 103 is extended away from the pump arm 31 toward the patient.

If desired, the support device 10 may be placed on an infusion pole by inserting a hook thereof (not shown) through strap 109. Alternatively, legs 20 may be rotated to its extended position and the support device 10 may be rested on its base 23 on a horizontal surface such as a table or the like. Finally, should the patient wish to be completely ambulatory, the support device may be inserted into a carrying case 90 with the infusion tube 103 extending out of the opening 123 to be attached to the patient.

Fluid delivery sets of the type having the flexible bag 49 are attached to the support device 10 in a manner similar to that described above with respect to the rigid bottle type fluid infusion set 16, except that the lid clamp 17 is moved to the desired extension position, and the lid 48 of the flexible bag 49 is inserted into the lid clamp 17 and securely clamped in place.

Similarly, fluid infusion sets 16 which include a burette type container can be positioned in the support device 10 in a manner similar to that described above with respect to the rigid bottle 47 in Figures 5-7, with the burette being placed between the bracket arms 62 of the saddle bracket 21.

With each type of fluid infusion set, if desired or necessary, the strap 19 may be used to secure the container in the container compartment 13. Although not shown, other standard fluid sets 16, such as spike sets, etc. can be similarly used with the support device 10 of the present invention.

As shown in Figure 43, the electrical connector cable 139 includes an elongated electrical cable 146 having a first connector 140 on one end thereof adapted for attachment to the ambulatory support device 10 and simultaneously to the infusion pump 30 when mounted in the ambulatory support device 10, and a second connector 141 on the opposite end thereof adapted for attachment to a charger unit (not shown).

As best shown in Figures 44-46, the first connector 140 includes a primary locking member 142 which extends from the connector housing 147 in a direction which is opposite the direction of extension of the cable 146 from the housing 147. An electrical connection member 145 also extends from the housing 147 and is directed parallel with the primary locking member 142, and includes an insertion tab 148 positioned in a slightly spaced apart relationship with the primary locking member 142.

The primary locking member 142 is formed of an extension arm 149 which extends away from the housing 147. A pair of resilient locking fingers 143 extend along opposing sides of the extension 149 in a co-planar relationship therewith. The fingers 143 are integrally formed with the extension 149 at hinging points 150 and extends generally parallel with the extension 149 in the direction of the housing 147.

Each resilient locking finger 143 is formed to include a locking surface 144 thereon which is designed to engage and lock behind detents located within a connector port in the support device in the manner as will be explained momentarily.

As shown in Figure 47, the electrical connection member 145 of the first connector 140 includes a series of openings 151 formed in the insertion tab 148 thereof which are adapted to receive a similarly oriented series of electrical pins (not shown) extending from the infusion pump 30. The openings 151 lead directly into housing 147 of the connector wherein direct electrical connection thereof is made to the cable 146 in a well known manner, whereby insertion of electrical pins from the infusion pump 30 into openings 151 causes electrical connection between the pump 30 and the cable 146.

As shown in Figures 48-50, the second connector 141 of the present invention includes a housing 152 which is adapted to receive the cable 146 at one end thereof, and is formed with a charger unit connection port 153 on an opposing end thereof. As shown in dashed lines, a plurality of electrical pins 154 extend into the charger unit connection port 153. The shape of the port 153 and the orientation of the pins 154 therein are predetermined to facilitate electrical connection to a charger unit (not shown).

Referring now to Figures 51 and 52, the pump compartment 12 of the support device 10 has formed therein a connector port 155. The connector port 155 extends from the back surface 45 of the support device 10 into the pump compartment 12. The connector port 155 includes a connector insertion opening 156 formed from the back surface 45, and surrounded by an elevated ridge 157. One surface of the ridge 157 has a notch 158 formed therein in order to avoid interference between the ridge 157 and the cable 146 as the first connector 140 is inserted into the connector insertion opening 156.

As shown in Figure 53, the first connector 140 is attached to the support device 10 by inserting the first connector 140 into the connector insertion opening 156 until the housing 147 therein and primary locking member 142 thereof lie completely within the connector insertion opening 156, and the cable 146 passes through the notch 158. Once within the opening 156, the first connector 140 is locked into its operating position within the support frame 10 by moving the connector 140 in the direction of the pump compartment 12 as shown by arrow 161 on the connector housing 147 (see Figure 44).

Movement in the direction of the pump compartment 12 causes the primary locking member 142 to pass into the recess 167. During movement of the primary locking member 142 into the recess 167, the resilient locking fingers 143 are forced into contact with detents 159 and are forced to resiliently rotate about hinge points 150 in the direction of extension 149. When the primary locking member 142 is inserted a sufficient distance into recess 167, the locking surfaces 144 on the fingers 143 will move into position against detents 159 and hold the locking member 142 against subsequent removal from the recess 167.

To remove the first connector 140 from the connector port 155, a user must perform a two step operation, including first, squeezing end surfaces 168 of the locking fingers 143 until they contact the extension 149, and then second, moving the first connector 140 away from the pump compartment 12 in the direction of arrow 162 (see Figure 44) until the primary locking member 142 is completely withdrawn from the recess 167. Once in this position, the first connector 140 can be removed from the connector insertion opening 156.

As best shown in Figures 52 and 53, when the first connector 140 is locked in position within the connector port 155, the primary locking member 142 is positioned flush with the rear surface 160 of the pump compartment 12 and the electrical connection member 145 protrudes into the pump compartment 12 in a direction parallel to the rear surface 160 and at a location spaced slightly apart therefrom. In this position, the electrical connection member 145 is placed for automatic connection with the infusion pump 30 whenever the infusion pump 30 is properly inserted into the pump compartment 12.

The second connector 141 of the connector cable 139 is attachable to a charger unit in a conventional manner. If desired, as shown in Figures 49 and 50, alignment ridges 163 may be positioned on the back surface 164 of the second connector 141 in order to facilitate insertion of the second connector 141 into the charging unit. An example of a charging unit adaptable for connection with the second connector 141 of the present invention is shown and described in the U.S. Patent No. 5,057,081 issued to Sunderland, on October 15, 1991.

It will be apparent from the foregoing, while particular embodiments of the invention have been illustrated and described, various modifications can be made thereto without departing from the scope of the present invention. Accordingly, it is not intended that the invention be limited, except as by the appended claims.

## Claims

1. A support device (10) for a fluid delivery system including a fluid delivery set (16) and a pump (30), the fluid delivery set (16) including a fluid container (47, 49) and a tube (98) for connection to the pump (30), said device (10) comprising
a body means (11) having:
first holding means (12) for holding the pump (30),
second holding means (13) for holding the container,
and characterised by
means (14) for preventing kinking or occlusion of the tube between the container and the pump.

2. A support device (10) according to claim 1 wherein said first holding means comprises a first compartment means.

3. A support device (10) according to claim 1 or claim 2 wherein said second holding means (13) comprises a second compartment means.

4. A support device according to claim 2 wherein said second holding means is adjustable.

5. A support device (10) according to any preceding claim wherein said means (14) for preventing kinking or occlusions of the tube (98) includes a third compartment means (14) in said body means (11) for holding the tube (98), said third compartment means (14) substantially enclosing the majority of the length of the tube (98) between the container (47) and the pump (30).

6. A support device (10) according to claim 5 wherein said body means (11) includes a fourth compartment means (15), whereby, a pinch clamp (79) included on tube (98) of the fluid delivery set (16) may be located within said fourth compartment means (15) when the tube (98) is properly located within said third compartment means (14).

7. A support device (10) according to claim 5 or claim 6 wherein said third compartment means (14) is a channel (14) extending around a substantial portion of a circumference of said body means (11).

8. A support device according to claim 7 wherein said channel (14) is generally U-shaped.

9. A support device according to claim 7 or claim 8 wherein said channel (14) forms an elongated opening through which the tube (98) can be inserted, and said opening includes means (113) for resiliently deforming the tube (98) as it passes into said channel (14).

10. A support device (10) according to claim 9 wherein said channel (14) forms an inlet opening (41) adjacent a first end of said elongated opening, and an exit opening (46) adjacent a second end of said elongated opening, said inlet opening (41) being located on said circumference of said body means (11) so as to conveniently receive the tube (98) from the container (47, 49) when located in said second holding means (13), and said exit opening (46) being located on said circumference of said body means (11) adjacent said first holding means (12) so as to allow the tube (98) to pass directly from said channel (14) to the pump (30) when located in said first holding means (12).

11. A support device (10) according to claim 9 wherein said means (113) for resiliently deforming said tube is an elongated lip (113) formed along at least a portion of said elongated opening of said channel (14).

12. A support device (10) according to any preceding claim wherein said first holding means (12) includes locking means (25) for locking said pump (30) there within.

13. A support device (10) according to claim 12 wherein said locking means (25) includes a locking pin (32) attached to a resilient lever arm means (33), whereby said locking pin (32) can be moved from a first position in which it can lock the pump (30) in place within said first holding means (12) and a second position wherein said pump (30) can be removed from said first holding means (12), said movement from said first to said second position being accomplished in use by resilient displacement of said lever arm (33).

14. A support device (10) according to any preceding claim wherein said second holding means (13) is formed as a recess (13) within a front surface (39) of said body means (11), said recess (13) including a substantially flat bottom surface (60) against which a portion of the container (47) rests when properly placed in said second holding means (13).

15. A support device (10) according to any preceding claim wherein said body means (11) is formed of a rigid material.

16. A support device (10) according to claim 15 wherein said body means (11) maintains said first holding means (12), said second holding means (13) and said means (14) for preventing kinking or occlusion of the tube (98), in fixed spaced relationship relative to each other.

17. A support device (10) according to claim 4 wherein said means (17, 19, 21) for holding the container within said second holding means includes strap means (19) attached to said body means (11) and operable to partially surround a container (47).

18. A support device (10) according to claim 4 wherein said means (17, 19, 21) for holding the container (49) in said second holding means (13) includes means (17) for securing a substantial portion of a mouth and lid (48) of the container (49) in relatively fixed position relative to said body means (11).

19. A support device (10) according to claim 18 wherein said means (17, 19, 21) for securing the mouth and lid of the container includes a U-shaped bracket means (21) adapted to receive the neck (68) and lid (115) of the container (47) therein when the container (47) is properly positioned in said second holding means (13).

20. A support device (10) according to claim 18 or claim 19 wherein said means (17) for securing the mouth and lid (48) of the container (49) includes a clamp means (50, 52) attached to said body means (11) for substantially surrounding the mouth and lid (48) of the container (49).

21. A support device (10) according to claim 20 wherein said U-shaped bracket means (21) is located within said second holding means (13).

22. A support device (10) according to claim 20 or claim 21 wherein said U-shaped bracket means (21) includes a spring bar member (69) which operates to assist the U-shaped bracket means (21) to maintain a position generally perpendicular to a substantially flat bottom surface (60) of the second holding means.

23. A support device (10) according to claim 21 or claim 22 wherein said second holding means (13) is formed as a recess (13) including a substantially flat bottom surface (60) against which a portion of the container (47, 49) rests when properly placed in said second holding means (13), and said U-shaped bracket means (21) is mounted within said flat bottom surface (60) and is operable between a first position in which said U-shaped bracket means (21) is flush with said flat bottom surface (60), and a second position in which said U-shaped bracket means (21) is rotated approximately 90 degrees to be substantially perpendicular to said flat bottom surface (60).

24. A support device (10) according to any of claims 20-23 wherein said clamp means (50, 52) is mounted to an extension means (18) for adjusting the position of said clamp means (50, 52) relative to said body means (11).

25. A support device (10) according to claim 34 wherein said extension means (18) includes an extension locking member (77) for locking said clamp means (50, 52) in any one of a plurality of positions relative to said body means (11) in order to accommodate various sizes of containers (49).

26. A support device (10) according to claim 25 wherein said extension locking member (77) is attached to said clamp means (50, 52) by at least one extension rod (72).

27. A support device (10) according to claim 26 wherein said at least one extension rod (72) is mounted in at least one generally tubular channel (73) formed by said body means (11) for slidable movement relative to said body means (11).

28. A support device (10) according to any preceding claim further including leg means (20) attachable to said body means (11) and rotatable between a first position in which said leg means (20) are oriented flush with a front surface (39) of said body means (11), and a second position in which said leg means (20) are oriented flush with a base surface (23) of said body means (11) and perpendicular to said front surface (39).

29. A support device (10) according to any of claims 1 - 27 further including leg means (20) attachable to said body means (11) and rotatable between a first position in which said leg means (20) is located within said body means (11) and flush with a front surface (39) of said body means, and a second position in which said leg means (20) is oriented substantially perpendicularly to said front portion (39) of said body means (11).

30. A support device (10) according to claim 29 wherein said leg means (20) is a single elongated generally flat member (20) including first and second extensions (165,166) and mounted for rotation within a base (23) of said body means (11), whereby, rotation of said leg means (20) to said
second position causes said first extension (165) to extend away from said front portion (39) and said second extension (166) to extend away from a back position (45) of said body means (11).

31. A support device (10) according to any of claims 28 to 30, wherein said leg means (20) includes locking means (11) for maintaining said leg means (20) in said second position.

32. A support device (10) according to any preceding claim wherein said body means (11) maintains said first holding means (12), said second holding means (13) and said means (14) for preventing kinking or occlusion of the tube (98), in fixed spaced relationship relative to each other.

33. A support device (10) according to any preceding claim and further including case means (90) for enclosing said body means (11) with the fluid delivery set (16) and pump (30) attached thereto.

34. A support device (10) according to claim 33 wherein said case means (90) forms a first opening (94) through which the container (47, 49) can be viewed when located within said case means (90), and a second opening (91) through which said pump (30) can be viewed when located within said case means (90).

35. A support device (10) according to claim 34 wherein said case means (90) further includes removable covering means (92, 97) for covering said first and second opening (94, 91) of said case means (90).

36. A support device (10) according to claim 35 wherein said covering means (97) for removable covering said second opening further includes a rigid stiffening member (127) therein.

37. A support device (10) according to any of claims 34 to 36 wherein said case means further includes a third opening through which said body means (11) can be inserted and a cover means (88) for securely closing said third opening.

38. A support device (10) according to any of claims 33-37 wherein said case means (90) is formed substantially of two similar halves reversibly separable by a fastening member (124) whereby, said case means (90) may be opened in clam-shell fashion to allow insertion of said body means (11) and subsequently reclosed by said fastening member (124).

39. A support device (10) according to any of claims 33-38 wherein said case means (90) for enclosing said body means (11) includes an extension means (129) for modifying the interior volume of the case means (90).

40. A support device (10) according to claim 39 wherein said extension means (129) is located on said case means (90) so as to accommodate at least a portion of said second holding means (13) of said body means (11).

41. A support device (10) according to claim 39 or claim 40 wherein said extension means (129) is formed of a front side flap (112), a back side flap (126), and a central flap (117), and said extension means (129) is capable of being formed into a non-extended position in which said front side flap (112) and said back side flap (126) are held adjacent said case means (90) in a non-extended position by said central flap (117), and an extended position in which said front side flap (112) and said back side flap (126) are extended away from said case means (90) in parallel relationship to each other and said central flap (117) passes around the perimeter of each of said front side flap (112) and back side flap (126) to form an enclosed extension of said case means (90).

42. A support device (10) according to any preceding claim and further including means (86, 87) for adjusting operation of the pump (30) according to the distance between the fluid container (47, 49) and the pump (30).

43. A support device (10) according to claim 42 wherein said means (86, 87) for adjusting includes a sensor member (88) on one of said support device (10) for the pump (30) and a sensed member (86) on the other of said support device (10) or the pump (30).

44. A support device (10) according to claim 43 wherein said means (86, 87) for adjusting operates only when said sensed member (86) is detected by said sensor member (87).

45. A support device (10) according to claim 43 or claim 44 wherein said sensed member (86) is a magnet and said sensor member (87) is a magnetic field sensitive sensor.

46. A support device (10) according to any preceding claim further including means (86, 87) for adjusting the operation of the pump (30) according to fluid pressure within the fluid delivery set (16).

47. A support device (10) according to claim 46 wherein said means (86, 87) for adjusting indirectly measures fluid pressure within the fluid delivery set (16).

48. A support device (10) according to any preceding claim and further including a connector cable (139) for electrical connection between a charger unit and the pump (30), said connector cable (139) comprising:
an electrical cable means (146) having a first end and a second end;
a first connector means (140) attached to said first end of said electrical cable means (146), said first connector means (140) including an electrical connection means (148) for electrical connection between said electrical cable means (146) and the pump (30), and a primary locking means (149) for locking said first connector (140) to said support device (10); and
means (141) for attaching said second end of said electrical cable means (146) to the charger unit.

49. A support device (10) according to claim 48 wherein said first connector means (140) includes a housing (147) and said primary locking means (149) includes an extension means (142) which extends from said housing (147) and includes at least one resilient finger means (143) having at least one locking surface (144) thereon, whereby, said first connector means (140) is locked to said support device (10) by resiliently deforming said resilient finger means (143) within said support device (10) until said locking surface (144) on said resilient finger means (143) contacts said support device (10).

50. A support device (10) according to claim 49 wherein said primary locking means (149) includes a pair of resilient finger means (143) each of said resilient finger means (143) being integrally connected with said extension means (142) by a resilient hinge (150).

51. A support device (10) according to any of claims 48-50 wherein said electrical connection means (158) is in electrical connection with the pump (30) whenever the pump (30) is properly mounted to said support device (10) and said first connector (140) is locked to said support device (10).

## Patentansprüche

1. Trägervorrichtung (10) für ein Fluidabgabesystem mit einem Fluidabgabeblock (16) und einer Pumpe (30), wobei der Fluidabgabeblock (16) einen Fluidbehälter (47, 49) und einen Schlauch (98) für eine Verbindung mit der Pumpe (30) aufweist und die Vorrichtung (10) eine Körpereinrichtung (11) mit einer ersten Halteeinrichtung (12) zum Halten der Pumpe (30) und mit einer zweiten Halteeinrichtung (13) zum Halten des Behälters hat, gekennzeichnet durch Einrichtungen (14) zum Unterbinden eines Knickens oder Verschließens des Schlauchs zwischen dem Behälter und der Pumpe.

2. Trägervorrichtung (10) nach Anspruch 1, bei welcher die erste Halteeinrichtung eine erste Kammereinrichtung aufweist.

3. Trägervorrichtung (10) nach Anspruch 1 oder Anspruch 2, bei welcher die zweite Halteeinrichtung (13) eine zweite Kammereinrichtung aufweist.

4. Trägervorrichtung nach Anspruch 2, bei welcher die zweite Halteeinrichtung einstellbar ist.

5. Trägervorrichtung (10) nach einem vorhergehenden Anspruch, bei welcher die Einrichtung (14) zur Unterbindung eines Knickens oder Verschließens des Schlauchs (98) eine dritte Kammereinrichtung (14) in der Körpereinrichtung (11) zum Halten des Schlauchs (98) aufweist, wobei die dritte Kammereinrichtung (14) im wesentlichen den größten Teil der Länge des Schlauchs (98) zwischen dem Behälter (47) und der Pumpe (30) umschließt.

6. Trägervorrichtung (10) nach Anspruch 5, bei welcher die Körpereinrichtung (11) eine vierte Kammereinrichtung (15) hat, wodurch eine auf dem Schlauch (98) des Fluidabgabeblocks (16) vorgesehene Schlauchklemme (79) in der vierten Kammereinrichtung (15) positioniert werden kann, wenn der Schlauch (98) richtig in der dritten Kammereinrichtung (14) positioniert ist.

7. Trägervorrichtung (10) nach Anspruch 5 oder Anspruch 6, bei welcher die dritte Kammereinrichtung (14) ein Kanal (14) ist, der sich um einen wesentlichen Abschnitt eines Umfangs der Körpereinrichtung (11) erstreckt.

8. Trägervorrichtung nach Anspruch 7, bei welcher der Kanal (14) insgesamt U-förmig ist.

9. Trägervorrichtung nach Anspruch 7 oder Anspruch 8, bei welcher der Kanal (14) eine langgestreckte Öffnung bildet, durch welche der Schlauch (98) eingeführt werden kann, wobei die Öffnung eine Einrichtung (113) zum elastischen Verformen des Schlauchs (98) aufweist, wenn er in den Kanal (14) hineingeht.

10. Trägervorrichtung (10) nach Anspruch 9, bei welcher der Kanal (14) eine Eintrittsöffnung (41) angrenzend an ein erstes Ende der langgestreckten Öffnung und eine Austrittsöffnung (46) angrenzend an ein zweites Ende der langgestreckten Öffnung bildet, wobei die Eintrittsöffnung (41) am Umfang der Körpereinrichtung (11) so angeordnet ist, daß sie den Schlauch (98) von dem Behälter (47, 49) in zweckmäßiger Weise bei der Positionierung in der zweiten Halteeinrichtung (13) aufnimmt und die Austrittsöffnung (46) an dem Umfang der Körpereinrichtung (11) angrenzend an die erste Halteeinrichtung (12) angeordnet ist, so daß der Schlauch (98) direkt von dem Kanal (14) zur Pumpe (30) gehen kann, wenn er in der ersten Halteeinrichtung (12) angeordnet ist.

11. Trägervorrichtung (10) nach Anspruch 9, bei welcher die Einrichtung (113) zum elastischen Verformen des Schlauchs eine langgestreckte Lippe (113) ist, die längs wenigstens eines Teils der langgestreckten Öffnung des Kanals (14) ausgebildet ist.

12. Trägervorrichtung (10) nach einem vorhergehenden Anspruch, bei welcher die erste Halteeinrichtung (12) eine Arretiereinrichtung (25) zum Arretieren der Pumpe (30) in ihr aufweist.

13. Trägervorrichtung (10) nach Anspruch 12, bei welcher die Arretiereinrichtung (25) einen Arretierstift (32) hat, der an einer elastischen Hebelarmeinrichtung (33) festgelegt ist, wodurch der Arretierstift (32) aus einer ersten Position, in der er die Pumpe (30) an Ort und Stelle in der ersten Halteeinrichtung (13) arretieren kann, und einer zweiten Stellung, in der die Pumpe (30) aus der ersten Halteeinrichtung (12) entfernt werden kann, bewegt werden kann, wobei die Bewegung aus der ersten in die zweite Stellung in Gebrauch durch elastisches Verschieben des Hebelarms (33) erreicht wird.

14. Trägervorrichtung (10) nach einem vorhergehenden Anspruch, bei welcher die zweite Halteeinrichtung (13) als eine Aussparung (13) in einer Vorderfläche (39) der Körpereinrichtung (11) ausgebildet ist, wobei die Aussparung (13) eine im wesentlichen ebene Bodenfläche (60) hat, auf der ein Abschnitt des Behälters (47) ruht, wenn er in der zweiten Halteinrichtung (13) richtig positioniert ist.

15. Trägervorrichtung (10) nach einem vorhergehenden Anspruch, bei welcher die Körpereinrichtung (11) aus einem starren Material hergestellt ist.

16. Trägervorrichtung (10) nach Anspruch 15, bei welcher die Körpereinrichtung (11) die erste Halteeinrichtung (12), die zweite Halteeinrichtung (13) und die Einrichtung (14) zur Unterbindung eines Knickens oder Verschließens des Schlauchs (98) in einer festgelegten Abstandsbeziehung zueinander hält.

17. Trägervorrichtung (10) nach Anspruch 4, bei welcher die Einrichtungen (17, 19, 21) zum Halten des Behälters in der zweiten Halteeinrichtung eine Bandeinrichtung (19) einschließen, die an der Körpereinrichtung (11) befestigt ist und so betätigbar ist, daß sie einen Behälter (47) teilweise umgibt.

18. Trägervorrichtung (10) nach Anspruch 4, bei welcher die Einrichtungen (17, 19, 21) zum Halten des Behälters (49) in der zweiten Halteeinrichtung (13) eine Einrichtung (17) zum Festlegen eines wesentlichen Teils einer Öffnung und eines Deckels (48) des Behälters (49) in einer relativ festgelegten Position bezüglich der Körpereinrichtung (11) einschließen.

19. Trägervorrichtung (10) nach Anspruch 18, bei welcher die Einrichtungen (17, 19, 21) zum Festlegen der Öffnung und des Deckels des Behälters eine U-förmige Bügeleinrichtung (21) aufweisen, die den Hals (68) und den Deckel (115) des Behälters (47) darin aufnehmen können, wenn der Behälter (47) richtig in der zweiten Halteeinrichtung (13) positioniert ist.

20. Trägervorrichtung (10) nach Anspruch 18 oder Anspruch 19, bei welcher die Einrichtung (17) zum Festlegen der Öffnung und des Deckels (48) des Behälters (49) eine Klemmeinrichtung (50, 52) aufweist, die an der Körpereinrichtung (11) für ein wesentliches Umschließen der Öffnung und des Deckels (48) des Behälters (49) befestigt ist.

21. Trägervorrichtung (10) nach Anspruch 20, bei welcher die U-förmige Bügeleinrichtung (21) in der zweiten Halteeinrichtung (13) positioniert ist.

22. Trägervorrichtung (10) nach Anspruch 20 oder Anspruch 21, bei welcher die U-förmige Bügeleinrichtung (21) ein Federstabelement (69) aufweist, das so wirkt, daß es die U-förmige Bügeleinrichtung (21) unterstützt, eine Lage insgesamt senkrecht zu einer im wesentlichen ebenen Bodenfläche (60) der zweiten Halteeinrichtung beizubehalten.

23. Trägervorrichtung (10) nach Anspruch 21 oder Anspruch 22, bei welcher die zweite Halteeinrichtung (13) als eine Aussparung (13) mit einer im wesentlichen ebenen Bodenfläche (60) ausgebildet ist, auf der ein Abschnitt des Behälters (47, 49) ruht, wenn er richtig in der zweiten Halteeinrichtung (13) positioniert ist, und die U-förmige Bügeleinrichtung (21) in der ebenen Bodenfläche (60) angebracht und zwischen einer ersten Position, in welcher die U-förmige Bügeleinrichtung (21) bündig zu der ebenen Bodenfläche (60) ist, und einer zweiten Position betätigbar ist, in der die U-förmige Bügeleinrichtung (21) um annähernd 90° gedreht ist, so daß sie im wesentlichen senkrecht zu der ebenen Bodenfläche (60) ist.

24. Trägervorrichtung (10) nach einem der Ansprüche 20 bis 23, bei welcher die Klemmeinrichtung (50, 52) auf einer Fortsatzeinrichtung (18) zum Einstellen der Position der Klemmeinrichtung (50, 52) bezüglich der Körpereinrichtung (11) angebracht ist.

25. Trägervorrichtung (10) nach Anspruch 24, bei welcher die Fortsatzeinrichtung (18) ein Fortsatzarretierelement (77) zum Arretieren der Klemmeinrichtung (50, 52) in irgendeiner aus einer Vielzahl von Positionen bezüglich der Körpereinrichtung (11) aufweist, um verschiedene Größen von Behältern (49) aufzunehmen.

26. Trägervorrichtung (10) nach Anspruch 25, bei welcher die das Fortsatzarretierelement (77) an der Klemmeinrichtung (50, 52) durch wenigstens eine Verlängerungsstange (72) festgelegt ist.

27. Trägervorrichtung (10) nach Anspruch 26, bei welcher die wenigstens eine Verlängerungsstange (72) in wenigstens einem insgesamt rohrförmigen, von der Körpereinrichtung (11) gebildeten Kanal (73) für eine Gleitverschiebebewegung relativ zur Körpereinrichtung (11) angeordnet ist.

28. Trägervorrichtung (10) nach einem vorhergehenden Anspruch, welche weiterhin eine Fußeinrichtung (20) aufweist, die an der Körpereinrichtung (11) befestigbar und zwischen einer ersten Position, in welcher die Fußeinrichtung (20) bündig zu einer Frontfläche (39) der Körpereinrichtung (11) ausgerichtet ist, und einer zweiten Stellung drehbar ist, in welcher die Fußeinrichtung (20) bündig zu einer Basisfläche (23) der Körpereinrichtung (11) und senkrecht zu der Vorderfläche (39) ausgerichtet ist.

29. Trägervorrichtung (10) nach einem der Ansprüche 1 bis 27, welche weiterhin eine Fußeinrichtung (20) aufweist, die an der Körpereinrichtung (11) befestigbar ist und zwischen einer ersten Position, in der welcher die Fußeinrichtung (20) in der Körpereinrichtung (11) positioniert und bündig zu einer Vorderfläche (39) der Köpereinrichtung ist, und einer zweiten Position drehbar ist, in der die Fußeinrichtung (20) im wesentlichen senkrecht zu dem vorderen Abschnitt (39) der Körpereinrichtung (11) ausgerichtet ist.

30. Trägervorrichtung (10) nach Anspruch 29, bei welcher die Fußeinrichtung (20) ein einzelnes langgestrecktes, insgesamt ebenes Element (20) mit einem ersten Ansatz (165) und einem zweiten Ansatz (166) ist und für eine Drehung in einer Basis (23) der Körpereinrichtung (11) angeordnet ist, wodurch eine Drehung der Fußeinrichtung (20) zu der zweiten Position den ersten Ansatz (165) dazu bringt, sich von dem vorderen Abschnitt (39) weg zu erstrecken, und den zweiten Ansatz (166) veranlaßt, sich aus einer Rückposition (45) von der Körpereinrichtung (11) weg zu erstrecken.

31. Trägervorrichtung (10) nach einem der Ansprüche 28 bis 30, bei welcher die Fußeinrichtung (20) eine Arretiereinrichtung (11) zum Halten der Fußeinrichtung (20) in der zweiten Position aufweist.

32. Trägervorrichtung (10) nach einem vorhergehenden Anspruch, bei welcher die Körpereinrichtung (11) die erste Halteeinrichtung (12), die zweite Halteeinrichtung (13) und die Einrichtung (14) zum Unterbinden des Knickens oder Verschließens des Schlauchs (98) in einer festgelegten Abstandsbeziehung relativ zueinander hält.

33. Trägervorrichtung (10) nach einem vorhergehenden Anspruch, welche weiterhin eine Gehäuseeinrichtung (90) zum Einschließen der Körpereinrichtung (11) mit dem Fluidabgabeblock (16) und der daran befestigen Pumpe (30) aufweist.

34. Trägervorrichtung (10) nach Anspruch 33, bei welcher die Gehäuseeinrichtung (90) eine erste Öffnung (94), durch welche der Behälter (47, 49) gesehen werden kann, wenn er sich in der Gehäuseeinrichtung (90) befindet, und eine zweite Öffnung (91) hat, durch welche die Pumpe (30) gesehen werden kann, wenn sie sich in der Gehäuseeinrichtung (90) befindet.

35. Trägervorrichtung (10) nach Anspruch 34, bei welcher die Gehäuseeinrichtung (90) weiterhin entfernbare Abdeckeinrichtungen (92, 97) zum Abdecken der ersten und zweiten Öffnung (94, 91) der Gehäuseeinrichtung (90) aufweist.

36. Trägervorrichtung (10) nach Anspruch 35, bei welcher die Abdeckeinrichtung (97) zum entfernbaren Abdecken der zweiten Öffnung weiterhin darin ein starres Absteifelement (127) aufweist.

37. Trägervorrichtung (10) nach einem der Ansprüche 34 bis 36, bei welcher die Gehäuseeinrichtung weiterhin eine dritte Öffnung, durch welche die Körpereinrichtung (11) eingeführt werden kann, und eine Abdeckeinrichtung (88) zum festen Verschließen der dritten Öffnung aufweist.

38. Trägervorrichtung (10) nach einem der Ansprüche 33 bis 37, bei welcher die Gehäuseeinrichtung (90) aus im wesentlichen zwei gleichen Hälften gebildet wird, die durch ein Befestigungselement (124) reversibel trennbar sind, wodurch die Gehäuseeinrichtung (90) muschelartig geöffnet werden kann, um das Einführen der Körpereinrichtung (11) zu ermöglichen und anschließend wieder durch das Befestigungselement (124) geschlossen werden kann.

39. Trägervorrichtung (10) nach einem der Ansprüche 33 bis 38, bei welcher die Gehäuseeinrichtung (90) zum Einschließen der Körpereinrichtung (11) eine Fortsatzeinrichtung (129) zum Modifizieren des Innenraumvolumens der Gehäuseeinrichtung (90) aufweist.

40. Trägervorrichtung (10) nach Anspruch 39, bei welcher die Fortsatzeinrichtung (129) an der Gehäuseeinrichtung (90) so positioniert ist, daß sie wenigstens einen Teil der zweiten Halteeinrichtung (13) der Körpereinrichtung (11) aufnimmt.

41. Trägervorrichtung (10) nach Anspruch 39 oder Anspruch 40, bei welcher die Fortsatzeinrichtung (129) von einer vorderen Seitenklappe (112), einer hinteren Seitenklappe (126) und einer zentralen Klappe (117) gebildet wird und in der Lage ist, in eine nicht ausgefahrene Position, in der die vordere Seitenklappe (112) und die hintere Seitenklappe (126) angrenzend an die Gehäuseeinrichtung (90) in einer nicht ausgefahrenen Stellung durch die zentrale Klappe (117), und in eine ausgefahrene Position gebracht werden kann, in der die vordere Seitenklappe (112) und die hintere Seitenklappe (126) sich von der Gehäuseeinrichtung (90) in paralleler Beziehung zueinander weg erstrecken und die zentrale Klappe (117) um den Umfang jeder der vorderen Seitenklappe (112) und hinteren Seitenklappe (126) herumgeht, um einen umschlossenen Fortsatz der Gehäuseeinrichtung (90) zu bilden.

42. Trägervorrichtung (10) nach einem vorhergehenden Anspruch, welche weiterhin Einrichtungen (86, 87) zum Einstellen der Arbeitsweise der Pumpe (30) entsprechend dem Abstand zwischen dem Fluidbehälter (47, 49) und der Pumpe (30) aufweist.

43. Trägervorrichtung (10) nach Anspruch 42, bei welcher die Einrichtungen (86, 87) zum Einstellen ein Sensorelement (88) an einer Trägervorrichtung (10) für die Pumpe (30) und ein vom Sensor abgetastetes Element (86) an der anderen Trägervorrichtung (10) für die Pumpe (30) aufweist.

44. Trägervorrichtung (10) nach Anspruch 43, bei welcher die Einrichtungen (86, 87) zum Einstellen nur arbeiten, wenn das vom Sensor abgetastete Element (86) von dem Sensorelement (87) erfaßt wird.

45. Trägervorrichtung (10) nach Anspruch 43 oder Anspruch 44, bei welcher das vom Sensor abgetastete Element (86) ein Magnet und das Sensorelement (87) ein auf ein Magnetfeld ansprechender Sensor ist.

46. Trägervorrichtung (10) nach einem vorhergehenden Anspruch, welche weiterhin Einrichtungen (86, 87) zum Einstellen der Arbeitsweise der Pumpe (30) gemäß dem Fluiddruck in dem Fluidabgabeblock (16) aufweist.

47. Trägervorrichtung (10) nach Anspruch 46, bei welcher die Einrichtungen (86, 87) zum Einstellen indirekt den Fluiddruck in dem Fluidabgabeblock (16) messen.

48. Trägervorrichtung (10) nach einem vorhergehenden Anspruch, welche weiterhin ein Verbinderkabel (139) für die elektrische Verbindung zwischen einer Ladeeinheit und der Pumpe (30) aufweist und das Verbinderkabel (39) eine elektrische Kabeleinrichtung (146) mit einem ersten Ende und einem zweiten Ende, eine erste Verbindereinrichtung (140), die an dem ersten Ende der elektrischen Kabeleinrichtung (146) festgelegt ist, wobei die erste Verbindereinrichtung (140) eine elektrische Verbindungseinrichtung (148) für eine elektrische Verbindung zwischen der elektrischen Kabeleinrichtung (146) und der Pumpe (30) und eine primäre Arretiereinrichtung (149) zum Arretieren des ersten Verbinders (140) an der Trägervorrichtung (10) hat, und Einrichtungen (141) zum Festlegen des zweiten Endes der elektrischen Kabeleinrichtung (146) an der Ladeeinheit aufweist.

49. Trägervorrichtung (10) nach Anspruch 48, bei welcher die erste Verbindereinrichtung (140) ein Gehäuse (147) und die primäre Arretiereinrichtung (149) eine Fortsatzeinrichtung (142) aufweist, die sich von dem Gehäuse (147) aus erstreckt und wenigstens eine elastische Fingereinrichtung (143) umfaßt, auf der wenigstens eine Arretierfläche (144) vorhanden ist, wodurch die erste Verbindereinrichtung (140) verriegelt wird, um die Trägervorrichtung (10) durch elastisches Verformen der elastischen Fingereinrichtung (143) in der Trägervorrichtung (10) arretiert wird, bis die Arretierfläche (144) auf der elastischen Fingereinrichtung (143) die Trägervorrichtung (10) kontaktiert.

50. Trägervorrichtung (10) nach Anspruch 49, bei welcher die primäre Arretiereinrichtung (149) ein Paar von elastischen Fingereinrichtungen (143) sind, wobei jede elastische Fingereinrichtung (143) einstückig mit der Fortsatzeinrichtung (142) durch ein elastisches Scharnier (150) verbunden ist.

51. Trägervorrichtung (10) nach einem der Ansprüche 48 bis 50, bei welcher die elektrische Verbindungseinrichtung (158) in einer elektrischen Verbindung mit der Pumpe (30) immer dann steht, wenn die Pumpe (30) richtig an der Trägervorrichtung (10) angebracht und der erste Verbinder (140) an der Trägervorrichtung (10) arretiert ist.

## Revendications

1. Dispositif de support (10) pour un système de distribution de fluide comportant un appareil de distribution de fluide (16) et une pompe (30), l'appareil de distribution de fluide (16) comportant un récipient à fluide (47, 49) et un tube (98) de raccordement à la pompe (30), ce dispositif (10) comprenant
un corps (11) ayant :
un premier moyen de support (12) destiné à supporter la pompe (30) et
un deuxième moyen de support (13) destiné à supporter le récipient,
et étant caractérisé par
un moyen (14) destiné à empêcher le tortillement ou l'occlusion du tube entre le récipient et la pompe.

2. Dispositif de support (10) selon la revendication 1, dans lequel le premier moyen de support comprend un premier compartiment.

3. Dispositif de support (10) selon l'une des revendications 1 et 2, dans lequel le deuxième moyen de support (13) comprend un deuxième compartiment.

4. Dispositif de support (10) selon la revendication 2, dans lequel le deuxième moyen de support est réglable.

5. Dispositif de support (10) selon l'une des revendications précédentes, dans lequel le moyen (14) destiné à empêcher le tortillement ou l'occlusion du tube (98) comporte un troisième compartiment (14) prévu dans le corps (11) pour tenir le tube (98), ce troisième compartiment (14) enfermant pratiquement la majeure partie de la longueur du tube (98) entre le récipient (47) et la pompe (30).

6. Dispositif de support (10) selon la revendication 5, dans lequel le corps (11) comporte un quatrième compartiment (15), et une pince (79) prévue sur le tube (98) de l'appareil de distribution de fluide (16) peut être placée dans ce quatrième compartiment (15) lorsque le tube (98) est convenablement placé dans le troisième compartiment (14).

7. Dispositif de support (10) selon l'une des revendications 5 et 6, dans lequel le troisième compartiment (14) est un canal (14) s'étendant autour d'une partie importante d'une circonférence du corps (11).

8. Dispositif de support selon la revendication 7, dans lequel le canal (14) est de manière générale en forme de U.

9. Dispositif de support selon l'une des revendications 7 et 8, dans lequel le canal (14) forme une ouverture allongée par laquelle le tube (98) peut être introduit, et cette ouverture comporte un moyen (113) destiné à déformer élastiquement le tube (98) lorsqu'il entre dans le canal (14).

10. Dispositif de support (10) selon la revendication 9, dans lequel le canal (14) forme une ouverture d'entrée (41) près d'une première extrémité de l'ouverture allongée et une ouverture de sortie (46) près d'une deuxième extrémité de l'ouverture allongée, l'ouverture d'entrée (41) étant située sur ladite circonférence du corps (11) de façon à recevoir commodément le tube (98) du récipient (47, 49) lorsque celui-ci est placé dans le deuxième moyen de support (13), et l'ouverture de sortie (46) étant placée sur ladite circonférence du corps (11) près du premier moyen de support (12) de façon à permettre au tube (98) de passer directement du canal (14) à la pompe (30) lorsque celle-ci est placée dans le premier moyen de support (12).

11. Dispositif de support (10) selon la revendication 9, dans lequel le moyen (113) destiné à déformer élastiquement le tube est une lèvre allongée (113) formée le long d'au moins une partie de l'ouverture allongée du canal (14).

12. Dispositif de support (10) selon l'une des revendications précédentes, dans lequel le premier moyen de support (12) comporte un moyen de blocage (25) destiné à y bloquer la pompe (30).

13. Dispositif de support (10) selon la revendication 12, dans lequel le moyen de blocage (25) comporte une goupille de blocage (32) fixée à un bras de levier élastique (33), et cette goupille de blocage (32) peut être déplacée entre une première position dans laquelle elle peut bloquer la pompe (30) en place dans le premier moyen de support (12) et une deuxième position dans laquelle la pompe (30) peut être enlevée du premier moyen de support (12), ce mouvement de la première à la deuxième position étant accompli lors de l'utilisation par déplacement élastique du bras de levier (33).

14. Dispositif de support (10) selon l'une des revendications précédentes, dans lequel le deuxième moyen de support (13) est un creux (13) fait dans une surface avant (39) du corps (11), ce creux (13) ayant une surface de fond sensiblement plane (60) contre laquelle repose une partie du récipient (47) lorsque celui-ci est convenablement placé dans le deuxième moyen de support (13).

15. Dispositif de support (10) selon l'une des revendications précédentes, dans lequel le corps (11) est fait d'une matière rigide.

16. Dispositif de support (10) selon la revendication 15, dans lequel le corps (11) maintient le premier moyen de support (12), le deuxième moyen de support (13) et le moyen (14) destiné à empêcher le tortillement ou l'occlusion du tube (98) espacés de manière fixe les uns des autres.

17. Dispositif de support (10) selon la revendication 4, dans lequel les moyens (17, 19, 21) destinés à tenir le récipient dans le deuxième moyen de support comprennent une courroie (19) attachée au corps (11) et manoeuvrable pour entourer partiellement un récipient (47).

18. Dispositif de support (10) selon la revendication 4, dans lequel les moyens (17, 19, 21) destinés à tenir le récipient (49) dans le deuxième moyen de support (13) comprennent un moyen (17) de fixation d'une partie importante d'une bouche et d'un couvercle (48) du récipient (49) dans une position relativement fixe par rapport au corps (11).

19. Dispositif de support (10) selon la revendication 18, dans lequel les moyens (17, 19, 21) de fixation de la bouche et du couvercle du récipient comprennent un étrier en U (21) fait pour recevoir le col (68) et le couvercle (115) du récipient (47) lorsque celui-ci est convenablement placé dans le deuxième moyen de support (13).

20. Dispositif de support (10) selon l'une des revendications 18 et 19, dans lequel le moyen (17) de fixation de la bouche et du couvercle (48) du récipient (49) comprend un collier (50, 52) attaché au corps (11) destiné à pratiquement entourer la bouche et le couvercle (48) du récipient (49).

21. Dispositif de support (10) selon la revendication 20, dans lequel l'étrier en U (21) est placé à l'intérieur du deuxième moyen de support (13).

22. Dispositif de support (10) selon l'une des revendications 20 et 21, dans lequel l'étrier en U (21) comprend une barre à ressort (69) qui agit pour aider l'étrier en U (21) à conserver une position de manière générale perpendiculaire à une surface de fond sensiblement plane (60) du deuxième moyen de support.

23. Dispositif de support (10) selon l'une des revendications 21 et 22, dans lequel le deuxième moyen de support (13) est un creux (13) ayant une surface de fond sensiblement plane (60) contre laquelle repose une partie du récipient (47, 49) lorsque celui-ci est convenablement placé dans le deuxième moyen de support (13), et l'étrier en U (21) est monté à l'intérieur de cette surface de fond plane (60) et est manoeuvrable entre une première position dans laquelle il est à fleur de la surface de fond plane (60) et une deuxième position dans laquelle il est tourné d'environ 90 degrés pour être sensiblement perpendiculaire à la surface de fond plane (60).

24. Dispositif de support (10) selon l'une des revendications 20 à 23, dans lequel le collier (50, 52) est monté sur une rallonge (18) pour le réglage de sa position par rapport au corps (11).

25. Dispositif de support (10) selon la revendication 24, dans lequel la rallonge (18) comprend un élément de blocage de rallonge (77) pour le blocage du collier (50, 52) dans une quelconque d'une série de positions par rapport au corps (11) pour l'adaptation à des récipients (49) de diverses dimensions.

26. Dispositif de support (10) selon la revendication 25, dans lequel l'élément de blocage de rallonge (77) est attaché au collier (50, 52) par au moins une tige rallonge (72).

27. Dispositif de support (10) selon la revendication 26, dans lequel la tige rallonge (72) est montée ou les tiges rallonges (72) sont montées dans au moins un canal de manière générale tubulaire (73) formé par le corps (11) pour coulisser par rapport à celui-ci.

28. Dispositif de support (10) selon l'une des revendications précédentes, comprenant en outre un pied (20) pouvant être attaché au corps (11) et tourner entre une première position dans laquelle il est orienté de façon à être à fleur d'une surface avant (39) du corps (11) et une deuxième position dans laquelle il est orienté de façon à être à fleur d'une surface de base (23) du corps (11) et perpendiculaire à ladite surface avant (39).

29. Dispositif de support (10) selon l'une des revendications 1 à 27, comprenant en outre un pied (20) pouvant être attaché au corps (11) et tourner entre une première position dans laquelle il est situé à l'intérieur du corps (11) et à fleur d'une surface avant (39) de celui-ci et une deuxième position dans laquelle il est orienté sensiblement perpendiculairement à ladite partie avant (39) du corps (11).

30. Dispositif de support (10) selon la revendication 29, dans lequel le pied (20) est un élément unique plat de manière générale allongé (20) comportant une première et une deuxième rallonges (165, 166) et monté tournant dans un socle (23) du corps (11), la rotation du pied (20) vers la deuxième position faisant s'écarter la première rallonge (165) de la partie avant (39) et la deuxième rallonge (166) d'un endroit arrière (45) du corps (11).

31. Dispositif de support (10) selon l'une des revendications 28 à 30, dans lequel le pied (20) comporte un moyen de blocage (11) destiné à le maintenir dans la deuxième position.

32. Dispositif de support (10) selon l'une des revendications précédentes, dans lequel le corps (11) maintient le premier moyen de support (12), le deuxième moyen de support (13) et le moyen (14) destiné à empêcher le tortillement ou l'occlusion du tube (98) espacés de manière fixe les uns des autres.

33. Dispositif de support (10) selon l'une des revendications précédentes, comportant en outre une boîte (90) destinée à enfermer le corps (11) avec l'appareil de distribution de fluide (16) et la pompe (30) attachés à lui.

34. Dispositif de support (10) selon la revendication 33, dans lequel la boîte (90) forme une première ouverture (94) par laquelle le récipient (47, 49) est visible lorsqu'il est placé dans la boîte (90) et une deuxième ouverture (91) par laquelle la pompe (30) est visible lorsqu'elle est placée dans la boîte (90).

35. Dispositif de support (10) selon la revendication 34, dans lequel la boîte (90) comporte en outre des moyens de couverture amovibles (92, 97) destinés à couvrir sa première et sa deuxième ouvertures (94, 91).

36. Dispositif de support (10) selon la revendication 35, dans lequel le moyen de couverture amovible (97) destiné à couvrir la deuxième ouverture contient un élément rigide de renfort (127).

37. Dispositif de support (10) selon l'une des revendications 34 à 36, dans lequel la boîte comporte en outre une troisième ouverture par laquelle le corps (11) peut être introduit et un couvercle (88) destiné à fermer solidement cette troisième ouverture.

38. Dispositif de support (10) selon l'une des revendications 33 à 37, dans lequel la boîte (90) est formée sensiblement de deux moitiés semblables séparables de manière réversible par un élément d'attache (124), et peut être ouverte à la manière d'une coquille de peigne pour permettre l'introduction du corps (11) et ensuite refermée par l'élément d'attache (124).

39. Dispositif de support (10) selon l'une des revendications 33 à 38, dans lequel la boîte (90) destinée à enfermer le corps (11) comporte une rallonge (129) destinée à modifier son volume intérieur.

40. Dispositif de support (10) selon la revendication 39, dans lequel la rallonge (129) est placée sur la boîte (90) de façon à recevoir au moins une partie du deuxième moyen de support (13) du corps (11).

41. Dispositif de support (10) selon l'une des revendications 39 et 40, dans lequel la rallonge (129) est formée d'un volet latéral avant (112), d'un volet latéral arrière (126) et d'un volet central (117) et peut être mise dans un état non allongé dans lequel le volet latéral avant (112) et le volet latéral arrière (126) sont maintenus près de la boîte (90) dans une position non allongée par le volet central (117), et dans un état allongé dans lequel le volet latéral avant (112) et le volet latéral arrière (126) sont écartés de la boîte (90) et parallèles et le volet central (117) passe autour du périmètre de chacun du volet latéral avant (112) et du volet latéral arrière (126) pour former une rallonge fermée de la boîte (90).

42. Dispositif de support (10) selon l'une des revendications précédentes, comportant en outre un moyen (86, 87) de réglage du fonctionnement de la pompe (30) d'après la distance entre le récipient à fluide (47, 49) et la pompe (30).

43. Dispositif de support (10) selon la revendication 42, dans lequel le moyen de réglage (86, 87) comporte un élément détecteur (87) sur l'un du dispositif de support (10) et de la pompe (30) et un élément détecté (86) sur l'autre du dispositif de support (10) et de la pompe.

44. Dispositif de support (10) selon la revendication 43, dans lequel le moyen de réglage (86, 87) fonctionne seulement lorsque l'élément détecté (86) est détecté par l'élément détecteur (87).

45. Dispositif de support (10) selon l'une des revendications 43 et 44, dans lequel l'élément détecté (86) est un aimant et l'élément détecteur (87) est un détecteur sensible aux champs magnétiques.

46. Dispositif de support (10) selon l'une des revendications précédentes, comportant en outre un moyen (86, 87) de réglage du fonctionnement de la pompe (30) d'après la pression du fluide dans l'appareil de distribution de fluide (16).

47. Dispositif de support (10) selon la revendication 46, dans lequel le moyen de réglage (86, 87) mesure indirectement la pression du fluide dans l'appareil de distribution de fluide (16).

48. Dispositif de support (10) selon l'une des revendications précédentes, comportant en outre un câble connecteur (139) pour la connexion électrique entre un bloc chargeur et la pompe (30), ce câble connecteur (139) comprenant :
un câble électrique (146) ayant une première et une deuxième extrémités,
un premier connecteur (140) fixé à la première extrémité du câble électrique (146), ce premier connecteur (140) comportant un moyen de connexion électrique (148) pour la connexion électrique entre le câble électrique (146) et la pompe (30) et un moyen de verrouillage primaire (149) pour le verrouillage du premier connecteur (140) au dispositif de support (10), et
un moyen (141) de fixation de la deuxième extrémité du câble électrique (146) au bloc chargeur.

49. Dispositif de support (10) selon la revendication 48, dans lequel le premier connecteur (140) comporte un boîtier (147) et le moyen de verrrouillage primaire (149) comporte une rallonge (142) qui s'étend à partir du boîtier (147) et comporte au moins un doigt élastique (143) ayant au moins une surface de verrouillage (144), le premier connecteur (140) étant verrouillé au dispositif de support (10) par déformation élastique de ce doigt élastique (143) dans le dispositif de support (10) jusqu'à ce que la surface de verrouillage (144) du doigt élastique (143) vienne en contact avec le dispositif de support (10).

50. Dispositif de support (10) selon la revendication 49, dans lequel le moyen de verrouillage primaire (149) comporte deux doigts élastiques (143) qui sont joints chacun à la rallonge (142) par une charnière élastique (150).

51. Dispositif de support (10) selon l'une des revendications 48 à 50, dans lequel le moyen de connexion électrique (158) est relié électriquement à la pompe (30) chaque fois que celle-ci est convenablement montée sur le dispositif de support (10) et que le premier connecteur (140) est verrouillé au dispositif de support (10).
